(19) Europäisches Patentamt
European Patent Office
Office européen des brevets

(11) Publication number: **0 033 094**
**B1**

(12) **EUROPEAN PATENT SPECIFICATION**

(45) Date of publication of patent specification: **10.10.84**

(51) Int. Cl.$^3$: **C 07 D 471/04,** A 61 K 31/44

(21) Application number: **81100247.6**

(22) Date of filing: **15.01.81**

# (54) Imidazo(1,2-a)pyridines, processes for their preparation and pharmaceutical compositions containing them.

(30) Priority: **23.01.80 US 114473**

(43) Date of publication of application:
**05.08.81 Bulletin 81/31**

(45) Publication of the grant of the patent:
**10.10.84 Bulletin 84/41**

(84) Designated Contracting States:
**BE CH DE FR GB IT LI LU**

(56) References cited:
**GB-A-1 524 589**
**US-A-4 146 642**

(73) Proprietor: **SCHERING CORPORATION**
**2000 Galloping Hill Road**
**Kenilworth, New Jersey 07033 (US)**

(72) Inventor: **Bristol, James Arthur**
**1921 High Hollow Drive**
**Ann Arbor Michigan 48103 (US)**
Inventor: **Puchalski Chester**
**9 Locust Avenue**
**Dover New Jersey 07801 (US)**

(74) Representative: **Antony, Fritz, Dr. et al**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 033 094 B1

Courier Press, Leamington Spa, England.

**Description**

This invention relates to novel imidazo[1,2-a]pyridines, to processes for their preparation, to pharmaceutical compositions containing such compounds and to methods for preparing such compositions. Representative compounds have been found to be useful as anti-secretory and cytoprotective agents and accordingly may be used for relief of the symptoms of peptic ulcers and for treating gastric and duodenal ulcers.

The imdazo[1,2-a]pyridines of this invention are of the general formula

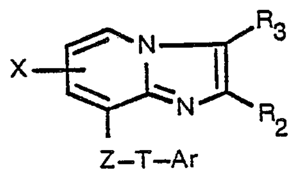

I

wherein

$R_2$ represents hydrogen, lower alkyl, trifluoromethyl, B—$CF_3$; B—Ar, Ar, B-halogen, $BOR_8$, B—$SO_{(n)}$-loweralkyl,

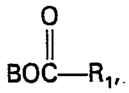 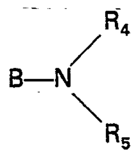 , $COOR_6$, 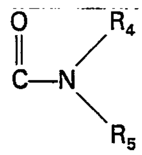 or B—CN;

$R_3$ represents loweralkyl, BCN, $BOR_8$,

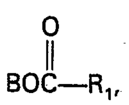 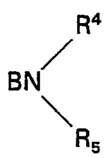 , 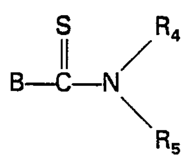 , 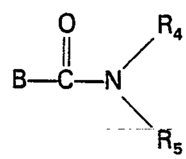 ,

B—$NO_2$, B-halogen or, provided $R_2$ is not hydrogen, hydrogen;

X is hydrogen, loweralkyl, halogen, hydroxy, trifluoromethyl or loweralkoxy

Z represents O, S, SO, $SO_2$, $NR_6$ or a single bond between the group T-Ar and position 8 of the imidazo[1,2-a]pyridine nucleus;

T represents a single bond between Z and Ar or a branched or straight alkylene group having 1 to 12 carbon atoms with no more than 5 binding carbon atoms between Z and Ar; and

Ar represents phenyl, pyridyl, thienyl, furanyl, imidazolyl or X'-, Y'- and/or Z'-substituted phenyl wherein each of X', Y' and Z' independently is hydrogen, halogen, loweralkyl, loweralkoxy, CN, $CF_3$,

OH, $NO_2$, 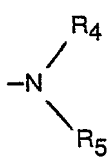 , $COOR_6$, 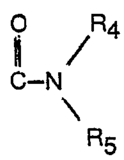 , 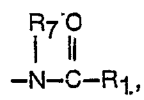 , 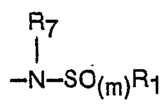 or $SO_{(m)}$—$R_1$,

with m being zero, one or two, whereby m represents two when $R_1$ represents

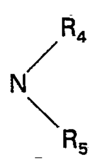

whereby in the above definitions

B is a straight or branched chain lower alkylene group;

n represents zero, one or two;

$R_4$ and $R_5$ each independently represent hydrogen, loweralkyl, lower-alkoxyloweralkyl, trifluoromethylloweralkyl, Ar-loweralkyl, Ar, or, when taken together with the nitrogen to which they are attached, form a 4- to 6-membered cyclic amino-group;

$R_1$ represents Ar, loweralkyl, 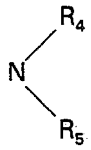 , 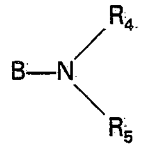 , or Ar-loweralkyl;

$R_6$ represents hydrogen, $C_1$- to $C_{12}$-alkyl, aryl- or an aralkyl group having up to 12 carbon atoms.

$R_7$ represents hydrogen or loweralkyl; and

$R_8$ represents hydrogen, loweralkyl, loweralkoxyloweralkyl, trifluoromethylloweralkyl, Ar-loweralkyl or Ar;

the 2,3-dihydro; 5,6,7,8-tetrahydro and 2,3,5,6,7,8-hexahydro analogues thereof and the pharmaceutically acceptable salts of such compounds.

As used herein, the term "halogen" includes fluoro, chloro, bromo and iodo, with fluoro and chloro being preferred; the term "lower", when applied to hydrocarbon groups, means includes straight and branched chain hydrocarbon groups having up to six carbon atoms such as methyl, ethyl, propyl, butyl, t-butyl, isopropyl, neopentyl, dimethylbutyl etc., whereby methyl and ethyl are preferred; the preferred definition of the group BCN, especially as $R_3$, is $—CH_2CN$; the term "pyridyl" includes the 2-, 3- and 4-isomers and their halogen- or loweralkyl-substituted analogues; the terms "thienyl" and "furanyl" include the 2- and 3-isomers and their halogen- and loweralkyl-substituted analogues; the term "imidazolyl" includes the 2- and 4-isomers and their halogen- and loweralkyl-substituted analogues. In those instances where X is other than hydrogen, such substituent may be at any of the positions 5-, 6- or 7- of the imidazo[1,2-a]pyridine nucleus. The preferred definitions of B and T are methylene or ethylene. The term "cyclic amino-group" is also meant to cover a morpholino-group.

A preferred subgroup of compounds of Formula I are those wherein

$R_2$ represents hydrogen, loweralkyl with 1 to 3 carbon atoms, $CH_2OH$, $CH_2CN$, $CH_2O—CO—CH_2N(CH_3)_2$ or 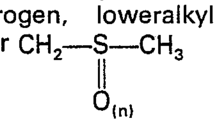

with n being zero, one or two;

$R_3$ is selected from hydrogen (provided $R_2$ is not hydrogen), methyl, $CH_2CN$,

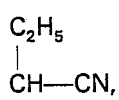, $CH_2OH$, 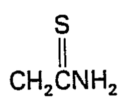

or $CH_2—O—CO—R_9$ with $R_9$ representing methyl, ethyl, propyl, isopropyl or t-butyl;

X represents hydrogen;

Z represents O, NH, SO or a single bond;

T represents a branched or straight lower alkylene group having up to 5 carbon atoms and

Ar is selected from phenyl, thienyl, pyridyl or a X′, Y′, Z′-substituted phenyl group wherein each of X′, Y′ and Z′ independently is selected from H, Cl, F, $CH_3$, t-butyl, $CF_3$, $OCH_3$ and CN.

From this broad group of preferred compounds, the following are of particular interest:

$R_2$ represents H, $CH_3$, $C_2H_5$, $i$-$C_3H_7$ or $CH_2OH$;

$R_3$ represents $CH_3$, $CH_2CN$, $CH_2OH$, $CH_2OCOCH_3$ or 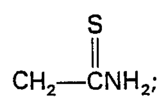;

X represents hydrogen;

Z represents O, NH or a single bond;

T represents $—CH_2—$, $—CH_2—CH_2—$, 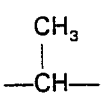 or $—CH_2—CH_2—CH_2—$; and

Ar represents phenyl, o- or p-fluorophenyl p-chlorophenyl or 2,4,6-trimethylphenyl.

A most preferred group of compounds is defined as follows:

$R_2$ represents $CH_3$,

$R_3$ represents $CH_2CN$ or $CH_2OH$,

Z represents O or NH,

T represents $—CH_2—$ and

Ar represents phenyl, o- or p-fluorophenyl; or 2,4,6-trimethylphenyl.

As mentioned above the compounds of formula I are useful in the treatment of ulcers. Compounds having a similar structure are known in the literature, e.g. from GB—A—1.524.589 and

US—A—4.146.642, the main difference being that the prior art compounds must have a carbonyl amino group

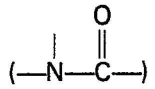

in position 2 or 3, a configuration which is not included in the present application. The prior art compounds are useful as fungicides and nowhere in the two citations is there any indication that the compounds may be used for treating ulcer conditions.

The following compounds are typical representatives of the preferred compounds of this invention:

1.) 2-Methyl-8-(2,4,6-trimethylbenzyloxy)-imidazo[1,2-a]pyridine;
2.) 8-(3,4-Dichlorobenzyloxy)-2-methyl-imidazo[1,2-a]pyridine;
3.) 8-(2-Fluorobenzyloxy)-2-methyl-imidazo[1,2-a]pyridine;
4.) 8-(4-Fluorobenzyloxy)-2-methyl-imidazo[1,2-a]pyridine;
5.) 2-Methyl-8-(2-phenylethyl)-imidazo[1,2-a]pyridine;
6.) 8-(4-Chlorobenzyloxy)-2-methyl-imidazo[1,2-a]pyridine;
7.) 8-Benzylsulfinyl-3-cyanomethyl-2-methyl-imidazo[1,2-a]pyridine;
8.) 3-Cyanomethyl-2-methyl-8-(2-thienyl-methoxy)-imidazo[1,2-a]pyridine;
9.) 3-Cyanomethyl-2-methyl-8-(2-pyridyl-methoxy)-imidazo[1,2-a]pyridine;
10.) 3-Cyanomethyl-2-methyl-8-(4-trifluoromethylbenzyloxy)-imidazo[1,2-a]pyridine;
11.) 3-Cyanomethyl-8-(3,4-dichlorobenzoyloxy)-2-methyl-imidazo[1,2-a]pyridine;
12.) 3-Cyanomethyl-8-(4-methoxybenzoyloxy)-2-methyl-imidazo[1,2-a]pyridine;
13.) 8-(4-t-butylbenzyloxy)-3-cyanomethyl-2-methyl-imidazo[1,2-a]pyridine;
14.) 8-Benzylthio-3-cyanomethyl-2-methyl-imidazo[1,2-a]pyridine;
15.) 3-Cyanomethyl-2-methyl-8-(3-trifluoromethylbenzoyloxy)-imidazo[1,2-a]pyridine;
16.) 8-(4-chlorobenzyloxy)-3-cyanomethyl-2-methyl-imidazo[1,2-a]pyridine;
17.) 8-Benzyloxy-2-methyl-imidazo[1,2-a]pyridine-3-methylpivaloate;
18.) 8-(3,4-Dichlorobenzoyloxy)-3-hydroxymethyl-2-methyl-imidazo[1,2-a]pyridine;
19.) 8-Benzyloxy-2-methyl-imidazo[1,2-a]pyridine-3-(2-butanonitrile);
20.) 8-(4-Chlorobenzyloxy)-3-hydroxymethyl-2-methyl-imidazo[1,2-a]pyridine;
21.) 8-Benzoyloxy-2-ethyl-imidazo[1,2-a]pyridine;
22.) 8-Benzyloxy-3-cyanomethyl-2-methylsulfinylmethyl-imidazo[1,2-a]pyridine;
23.) 8-Benzyloxy-3-cyanomethyl-2-methylthiomethyl-imidazo[1,2-a]pyridine;
24.) 8-Benzyloxy-3-cyanomethyl-imidazo[1,2-a]pyridine-2-methyl-N,N-dimethylglycinate;
25.) 8-(4-Cyanobenzyloxy)-3-cyanomethyl-2-methyl-imidazo[1,2-a]pyridine;
26.) 8-Benzyloxy-2-cyanomethyl-imidazo[1,2-a]pyridine;
27.) 8-Benzyloxy-3-cyanomethyl-imidazo[1,2-a]pyridine;
28.) 8-Benzyloxy-2-methyl-imidazo[1,2-a]pyridine-3-methylacetate;
29.) 8-Benzyloxy-3-cyanomethyl-2-hydroxymethyl-imidazo[1,2-a]pyridine;
30.) 3-Hydroxymethyl-2-methyl-8-(2-phenylethoxy)-imidazo[1,2-a]pyridine;
31.) 8-Benzyloxy-2,3-dimethyl-imidazo[1,2-a]pyridine;
32.) 3-Cyanomethyl-2-methyl-8-(2-phenylethoxy)-imidazo[1,2-a]pyridine;
33.) 3-Cyanomethyl-2-methyl-8-(1-phenylethoxy)-imidazo[1,2-a]pyridine;
34.) 3-Cyanomethyl-2-methyl-8-(2-phenylethyl)-imidazo[1,2-a]pyridine;
35.) 8-Benzyloxy-2-methyl-imidazo[1,2-a]pyridine-3-thioacetamide;
36.) 3-Hydroxymethyl-2-methyl-8-(2-phenylethyl)-imidazo[1,2-a]pyridine;
37.) 3-Cyanomethyl-2-methyl-8-(3-phenylpropoxy)-imidazo[1,2-a]pyridine;
38.) 8-Benzyloxy-3-cyanomethyl-2-isopropyl-imidazo[1,2-a]pyridine;
39.) 8-Benzyloxy-3-cyanomethyl-2-ethyl-imidazo[1,2-a]pyridine;
40.) 8-Benzylamino-2,3-dimethyl-imidazo[1,2-a]pyridine;
41.) 8-Benzyloxy-3-cyanomethyl-2-methyl-imidazo[1,2-a]pyridine;
42.) 8-Benzyloxy-3-hydroxymethyl-2-methyl-imidazo[1,2-a]pyridine;
43.) 3-Hydroxymethyl-8-(2-fluorobenzyloxy)-2-methyl-imidazo[1,2-a]pyridine;
44.) 8-(4-t-Butylbenzyloxy)-3-hydroxymethyl-2-methyl-imidazo[1,2-a]pyridine;
45.) 3-Cyanomethyl-8-(2-fluorobenzyloxy)-2-methyl-imidazo[1,2-a]pyridine;
46.) 3-Cyanomethyl-8-(4-fluorobenzyloxy)-2-methyl-imidazo[1,2-a]pyridine;
47.) 3-Cyanomethyl-2-methyl-8-(2,4,6-trimethylbenzyloxy)-imidazo[1,2-a]pyridine;
48.) 8-Benzylamino-3-cyanomethyl-2-methyl-imidazo[1,2-a]pyridine;
49.) 3-Cyanomethyl-2-methyl-8-(3-thienyl-methoxy)-imidazo[1,2-a]pyridine.

The compounds of this invention may be obtained by processes generally known for the preparation of compounds having a similar structure. The basic step consists in formation of the (substituted) imidazo[1,2-a]pyridine nucleus:

A: 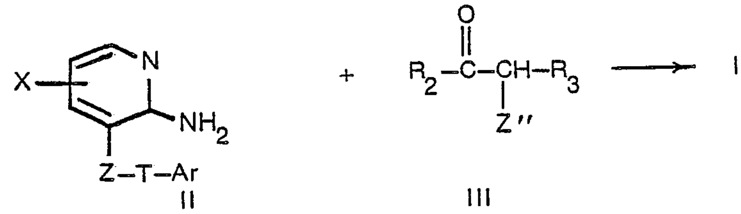

II III

In the above formulae II and III, $R_2$, $R_3$, X, Z, T and Ar are as defined hereinabove, and Z'' represents a good leaving group, preferably a reactive inorganic or organic ester group, such as halogen, tosyl, mesyl etc. Any free amino- or hydroxy groups present in $R_2$, $R_3$, X and Ar may advantageously be protected by a protecting group, which is subsequently removed. The reaction is preferably carried out by heating the reactants together (e.g. at reflux temperature) in an inert solvent. If in the above reaction a compound of Formula III wherein $R_2$ and $R_3$ are both hydrogen and Z'' is chlorine is used (chloroacetaldehyde), then $R_2$ and $R_3$ in the final compound are both hydrogen, i.e.

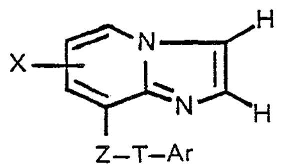

These compounds are intermediates useful in the preparation of compounds falling within the scope of this invention, e.g. as used in Step (ii) of process C hereinbelow:

If a compound of Formula III wherein $R_2$ is methyl, $R_3$ is hydrogen and Z'' is chlorine (chloracetone) is used, then a final compound of the Formula

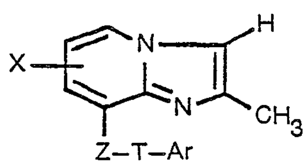

is obtained.

For preparing compounds wherein Z represents S, O, $NR_6$ or a single bond, the following process may be used

B: 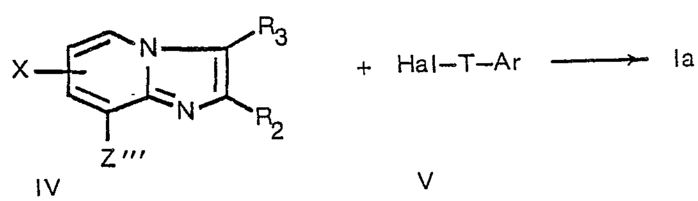

IV V

In the above Formulae IV and V Hal represents Br, Cl or J, Z''' represents hydrogen, halogen (Cl, Br, I), OH, SH or $NHR_6$ and X, $R_2$, $R_3$, T and Ar are as above defined.

The reactants are heated together under standard reaction conditions known from the preparation of simular compounds, e.g. in an inert solvent in the presence of a base. When Z''' represents halogen, a copper catalyst is preferably used. When Z''' represents OH, SH or $NHR_6$, the reaction may be carried out with or without such copper catalyst.

The starting compounds in the above reactions (A) and (B) are either known or may be obtained according to standard procedures, some of which are exemplified hereinafter.

As is obvious to anyone skilled in the art, numerous standard reactions may be applied for transferring one type of substituent $R_2$ and/or $R_3$ into another type. Thus, for example, for preparing compounds of Formula I wherein $R_3$ represents the group BCN, the following processes may be applied:

C. A compound of general Formula I wherein $R_2$, X, Z, T and Ar are as defined for Formula I but wherein $R_3$ is replaced by a group B X'' wherein B is as defined above and X'' represents a good leaving

group, is reacted with a metal cyanide to yield the desired product. Preferred leaving groups are $Si(CH_3)_2$, halogen, alkoxy, aryloxy, mesyl, tosyl, quaternary groups such as $\overset{\oplus}{N}(CH_3)_3 \cdot I^{\ominus}$ and quaternary groups wherein the quaternary ion is a non-nucleophilic counter ion such as $BF_4^{\ominus}$, $PF_6^{\ominus}$, $CF_3SO_3^{\ominus}$, $FSO_3^{\ominus}$, etc. Additionall one may carry out the displacement reaction in the presence of a crown ether. The preferred metal cyanides are alkali metal cyanides.

The reaction is carried out under standard conditions, e.g. by heating the reactants in an inert solvent, preferably dimethylformamide. When the quaternary anion is a non-nucleophilic counter ion, the reaction may also be carried out in an aqueous solvent.

The starting compounds of this process may be obtained by standard procedures, e.g. as illustrated below for the quaternary iodide salt:

(i) A compound of Formula II is reacted with a compound of Formula III wherein $R_3$ is hydrogen according to process A above.

(ii) The compound resulting from step (i), which is a compound of Formula I wherein $R_3$ is hydrogen, is further reacted with dimethylamine hydrochloride and paraformaldehyde by heating the reactants in methanol at reflux temperature.

(iii) The compound of step (ii) a compound of Formula I wherein $R_3$ represents $—CH_2N(CH_3)_2$ is reacted with $CH_3I$ to yield the desired methiodide.

D. A compound of Formula I wherein $R_2$, X, T, Z and Ar are as defined for Formula I and $R_3$ represents either of the groups — $BCONH_2$ and B—C—H
‖
N—OH

is subjected to a dehydration. The reaction is carried out by treating the starting compounds with a suitable dehydrating agent in an inert solvent, preferably at reduced temperatures. Preferred dehydrating agents are $(CF_3CO)_2O$ (in pyridine), $SeO_2$ (in trichloromethane), $POCl_3$, etc. The starting compounds may be obtained according to standard procedures, e.g. as indicated hereinabove and as exemplified hereinafter.

E. A compound of Formula I wherein $R_2$, X, T, Z and Ar are as defined for formula I and $R_3$ represents the group —B—C—H
‖
O

is treated with a suitable cyanocompound, e.g. Tosyl-$CH_2$—NC in the presence of potassium-*t*-butoxide whereby the formyl function is replaced by CN.

F. A compound of Formula I wherein $R_2$, X, T, Z and Ar are as defined for Formula I and $R_3$ represents the group —B COOR (ester) is treated with a suitable amine, e.g. dimethylaluminium-amine resulting in a compound where $R_3$ is —BCN.

G. A compound of Formula I wherein $R_2$, X, T, Z and Ar are as defined for Formula I and $R_3$ represents the group —B—CH—COOH
|
$NH_2$

is treated with NaOCl under standard conditions.

H. A compound of Formula I wherein $R_2$, X, T, Z and Ar are as defined for Formula I and $R_3$ represents a group B—$CH_2NO_2$ is subjected to a reductive dehydration, e.g. with $PCl_3$ and the like in pyridine to give the desired nitrile. [See J. Org. Chem. 42, 3956 (1977)].

I. A compound of Formula I wherein $R_2$, X, T, Z and Ar are as defined for Formula I and $R_3$ represents H is reacted with a compound of the Formula Hal-B CN wherein Hal is chlorine or bromine in the presence of a Lewis acid (e.g. aluminium chloride, zinc chloride, boron chloride etc.) or a phase transfer catalyst.

J. A compound of Formula I wherein $R_2$, X, T, Z and Ar are as defined for Formula I and $R_3$ represents the group —CH—CN
|
$SCH_3$

is subjected to a reduction, preferably with Raney-nickel whereby the —$SCH_3$— group is replaced by a hydrogen atom. The starting compound may be obtained by reacting a compound of Formula I wherein $R_3$ represents hydrogen with $CH_3$—S—CH(Cl)CN in the presence of a Friedel Crafts catalyst (e.g. $SnCl_4$, $TiCl_4$, $AlCl_3$, etc.).

In addition to modifying various $R_3$ groups into —B—CN groups as described in reactions (C) to (J) above, also other transformations may be carried out, e.g. as indicated below:

| Starting $R_3$ | Chemical Reaction | Resulting $R_3$ |
|---|---|---|
| $—BCOOC_2H_5$ | reduction ($LiAlH_4$) | $—BCH_2OH$ |
| $—CH_2CN$ | reaction with alkylhalide | alkyl<br>\|<br>$—CH—CN$ |
| $—BCN$ | reduction with $LiAlH_4$ | $—BCH_2NH_2$ |
| $—BCH_2NH_2$ | 1. reaction with methyliodide<br>2. followed by reaction with metal cyanide | $—B \cdot CH_2CN$ |
| $—BCN$ | saponification | $—BCOOH$ |
| $—BOH$ | reaction with NaH and $Cl \cdot CO \cdot N—(CH_3)_2$ | $—B—O.CO \cdot N(CH_3)_2$ |
| $—BCN$ | treatment with NaOH | $—BCONH_2$ |
| $—BCN$ | treatment with $H_2S$ | S<br>\|\|<br>$—B—C—NH_2$ |
| $—BOH$ | treatment with $SOCl_2$ | $—B \cdot Cl$ |
| $—BX''$<br>($X''$=leaving group, e.g. halogen) | treatment with $NO_2^{\ominus}$ | $—BNO_2$ |
| $—BX''$<br>$X''$=leaving group, e.g. halogen | reaction with $CH_3NO_2$ | $B \cdot CH_2NO_2$ |
| $—CHO$ | 1. reaction with $CH_3NO_2$ resulting in $CH=CHNO_2$<br>2. treatment with $NaBH_4$ | $CH_2CH_2NO_2$ |
| NHAc<br>\|<br>$—CH_2CH—COOH$ | basic hydrolysis | $NH_2$<br>\|<br>$—CH_2CH—COOH$ |

Equally the various possibilities of $R_2$ may, where appropriate, be transferred into other $R_2$-substituents by reactions such as those outlined for $R_3$ in the processes (C) to (J) and the table above.

Compounds of Formula I wherein T represents SO or $SO_2$ may be obtained by oxidizing the corresponding compound wherein T represents S according to procedures well known in the art.

It is also obvious to anyone skilled in the art that the sequence of certain reactions may be altered. Thus, for example, one may, in accordance with process (A) above first prepare a compound of the formula

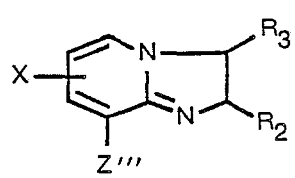

,

make the above described rearrangements within the groups $R_2$ and $R_3$ and then complete the molecule by carrying out process (B) above.

Example 1

8-Benzyloxy-2-methyl-imidazo[1,2-a]pyridine

*Step A:* Preparation of 2-amino-3-benzyloxypyridine.

In a 12 liter 3-neck round bottom flask equipped with a mechanical stirrer and thermomether there were placed 2.5 liters of 40% sodium hydroxide solution, 26.5 g of Adogen 464 (Reg. Trademark) and 2.5 liters of dichloromethane. To this vigorously stirred mixture was added 550 g of 2-amino-3-hydroxypyridine. The temperature was 38°C. The brown orange mixture was cooled to 25°C, and 677.5 g of benzylchloride was added in one portion and the mixture was allowed to separate into 2 phases. The lower aqueous phase was separated and diluted with 1 liter of ice:water. This solution was then extracted with dichloromethane (3 x 15 liters). The combined dichlormethane extracts were added to the original dichloromethane phase, washed with 1 liter of saturated sodium chloride solution and dried over potassium carbonate. The dichlormethane extract was filtered and concentrated on the rotary evaporator to an orange solid. This solid was dissolved in 1 liter of boiling absolute ethanol, and the solution was filtered. The filtrate was chilled, and the crystals that formed were filtered, washed with 500 ml of ethanol at −10°C, and dried at 50°C in a vacuum oven, to give the desired product.

In a similar manner, reaction of 2-amino-3-hydroxypyridine with the appropriate benzylhalide leads to the following aminopyridines:

2-amino-3-(4-methoxybenzyloxy)-pyridine,
2-amino-3-(3-trifluoromethylbenzyloxy)-pyridine,
2-amino-3-(4-chlorobenzyloxy)-pyridine,
2-amino-3-(2-fluorobenzyloxy)-pyridine,
2-amino-3-(4-*t*-butylbenzyloxy)-pyridine,
2-amino-3-(2-phenylethoxy)-pyridine and
2-amino-3-(3,4-dichlorobenzyloxy)-pyridine.

*Step B:* Preparation of title compound

Into a 12 liter 3 necked flask equipped with a mechanical stirrer and condenser, were placed 750 g of 2-amino-3-benzyloxy-pyridine (obtained according to Step A), 6.75 liters of absolute ethanol (one may also use methanol≈3 liters) and 360 ml of chloroacetone. The solution was heated under reflux for 4 hrs.

An additional 180 ml of chloroacetone was added and the dark solution was heated under reflux for 18 hrs.

The solvent was evaporated off and the residual dark oil was dissolved in 7 liters of water. The resulting solution was made strongly basic with 15% sodium hydroxide and the basified solution was extracted with several portions (4 x 1.5 liters) of dichloromethane. The extracts were combined and washed with brine and the washed extracts evaporated down to a dark gum which was boiled with 7 liters of diisopropyl ether. The solution was decanted from insoluble material through a glass wool plug, and the filtrate was chilled. The resulting crystals were filtered and washed with cold diisopropyl ether. Recrystallization provided the pure product of this example (m.p. 93—95°C).

Similarly, reaction of the appropriate 2-amino-3-arylalkyloxypyridine (which may be synthesized from 2-amino-3-hydroxypyridine according to the procedures in *Acta. Chem. Scand., 23,* 1791 (1969).) and chloroacetone leads to the following imidazo[1,2-a]pyridines:

8-(4-methoxybenzyloxy-2-methyl-imidazo[1,2-a]pyridine;
2-methyl-8-(3-trifluoromethylbenzyloxy)-imidazo[1,2-a]pyridine,
8-(4-fluorobenzyloxy)-2-methyl-imidazo[1,2-a]pyridine,
8-(3,4-dichlorobenzyloxy)-2-methyl-imidazo[1,2-a]pyridine; and
8-(4-chlorobenzyloxy)-2-methyl-imidazo[1,2-a]pyridine.

Example II

8-Benzyloxy-imidazo[1,2-a]pyridine-2-carboxylic acid ethylester

Ethyl bromopyruvate (19.8 g.) in 25 ml of 1,2-dimethoxyethane was added to 16.0 g. of 2-amino-3-benzyloxypyridine in 100 ml. of 1,2-dimethoxyethane over 0.25 hr. After 1 hr. the solid product which was formed was collected by filtration and washed with 1,2-dimethoxyethane and ether. The material was then heated under reflux in ethanol. Upon cooling the reaction was rendered basic with 22 ml. of 15% sodium hydroxide solution. The solid product was recrystallized from ethanol to yield 8-benzyloxy-imidazo[1,2-a]pyridine-2-carboxylic acid, ethyl ester (m.p. 163—167°C).

In the same manner, reaction of an appropriate amino-pyridine with ethyl bromopyruvate leads to the following compounds:

8-(4-methoxybenzyloxy)-imidazo[1,2-a]pyridine-2-carboxylic acid ethyl ester,
8-(3-trifluoromethylbenzyloxy)-imidazo[1,2-a]pyridine-2-carboxylic acid ethyl ester,
8-(4-fluorobenzyloxy)-imidazo[1,2-a]pyridine-2-carboxylic acid ethyl ester,
8-(3,4-dichlorobenzyloxy)-imidazo[1,2-a]pyridine-2-carboxylic acid ethyl ester and
8-(2-fluorobenzyloxy)-imidazo[1,2-a]pyridine-2-carboxylic acid ethyl ester.

Example III

8-Benzyloxy-3-cyanomethyl-2-methyl-imidazo[1,2-a]pyridine

*Step A:* Preparation of 3-chloro-4-oxopentanonitrile.

Into a 1-neck, 3-l round bottom flask there were placed 1 l diethylether ($Et_2O$) and 100 g 4-oxopentanonitrile. The magnetically stirred solution was cooled to 0—5°C, one drop $HCl/Et_2O$ added, and 185 ml 97% $SO_2Cl_2$ previously chilled to 5—10° was added all at once. The ice bath was removed, and the pale greenish-yellow solution was warmed to 20 ± 1°C over 5 min. by a hot water bath. The temperature was maintained at 20 ± 1°C by a cold water bath for 2½ hr. The pale yellow solution was evaporated on a rotary evaporator in a 30° water bath at 80 mm vacuum, and carefully watched. Near the end of solvent removal, the *instant* the near-colorless residue began to turn orange, the flask was removed *quickly* and diluted with 1 l cold $Et_2O$. One ml $SO_2Cl_2$ was added and stirred 15 min., and the orangish solution was diluted with 1 l cold $Et_2O$.

The ether solution was washed with 1 l cold saturated $NaHCO_3$-solution which was in turn extracted with 2 × 1/2 1 cold $CH_2Cl_2$. The $CH_2Cl_2$ was evaporated, the residue dissolved in 200 ml $Et_2O$, and added to the bicarbonate-washed ether solution. The ether was extracted by 2 × 1 l cold 10% $NaHSO_3$-solution and discarded. The bisulfite solution was cooled in an ice bath and 25% NaOH was added slowly to attain pH 7 (ca. 100 ml), 100 g $NaHCO_3$ was added to saturate the solution, and it was extracted with 5 × 1 l $CH_2Cl_2$; 25 g $K_2CO_3$ was added followed by extraction with 1 l $CH_2Cl_2$, and this was repeated with another 25 g $K_2CO_3$ — 1 l $CH_2Cl_2$. The combined extracts were dried over $MgSO_4$, and evaporated to leave a brown-orange oil, estimated from pmr to contain 3-Cl isomer, 5-Cl isomer, some unknown compounds and $Ch_2Cl_2$.

Distillation of this oil through a jacketed 15-cm Vigreux column at 0.3 mmHg gave 3-chloro-4-oxopentanonitrile (b.p. 83—93°).

*Step B:* Preparation of title compound

A solution of 0.86 g. of 3-chloro-4-oxopentanonitrile, 0.92 g. of 2-amino-3-phenylmethoxypyridine and 0.5 ml. of triethylamine in 15 ml. of ethanol was stirred for 16 hours, then concentrated to remove the triethylamine and ethanol. The residue was dissolved in methylene chlorid and washed with water. The methylene chloride was concentrated and the residual oil was dissolved in 20 ml. of boiling acetonitrile, filtered, cooled, and treated with hydrogen chloride/methanol with cooling to give 0.22 g. of the desired product (m.p. 163—166°C).

Example IV

8-(4-Fluorobenzyloxy)-2-methyl-imidazo[1,2-a]pyridine.

*Step A:* Preparation of 8-hydroxy-2-methyl-imidazo[1,2-a]pyridine.

A mixture of 2-amino-3-hydroxypyridine (1.000 g) and chloroacetone (845 g) in 7.57 liters of ethanol was heated under reflux for 20 hrs. Most of the ethanol was removed by distillation and the residual material was dissolved in 7.57 liters of water. This solution was extracted four times with 1.2 liter portions of dichloromethane. The combined extracts were washed with 1 liter of water and this was combined with the aqueous solution. This solution was then adjusted to pH 11.5—12 with 850 ml of 50% sodium hydroxide. This basic solution was extracted three times with 1.2 liters of dichloromethane. The combined dichloromethan extracts were washed with 1 liter of $H_2O$ and this was added to the basic solution. This solution was chilled in an ice bath and the pH adjusted to 7 with 1.45 liters of 6N hydrochloric acid. After standing overnight, the product was collected, washed with water, and dried. The product was recrystallized from dichloromethane/methanol to give the intermediate.

*Step B:* Preparation of the title compound

To a stirred suspension under nitrogen of 15.0 g 8-hydroxy-2-methyl-imidazo[1,2-a]pyridine (from Step A) in 150 ml dimethylformamide maintained at 0—5°C, was added 5.15 g 50% sodium hydride-oil dispersion in portions over 10 minutes. The mixture was stirred in the cold ½ hr. and a brown solution resulted. 15.3 g 4-Fluorobenzyl chloride was added, and the solution was heated on a steam bath for ½ hr. The solution was poured into an ice bath and after ½ hour it became a brown solution. The solution was poured into 1.5 liters ice/water with vigorous stirring and the precipitate was separated by suction filtration, washed, and dried to give 8-(4-flurobenzyloxy)-2-methyl-imidazo[1,2-a]pyridine (m.p. 137°C).

By using appropriately substituted benzyl chlorides in the above reaction the following compounds are obtained:

8-(2-fluorobenzyloxy)-2-methyl-imidazo[1,2-a]pyridine,
8-(4-chlorobenzyloxy)-2-methyl-imidazo[1,2-a]pyridine,
8-(3,4-dichlorobenzyloxy)-2-methyl-imidazo[1,2-a]pyridine,
2-methyl-8-(3-trifluoromethylbenzyloxy-imidazo[1,2-a]pyridine,
2-methyl-8-(4-trifluoromethylbenzyloxy)-imidazo[1,2-a]pyridine,
8-(2,6-dimethylbenzyloxy)-2-methyl-imidazo[1,2-a]pyridine,
8-(4-methoxybenzyloxy)-2-methyl-imidazo[1,2-a]pyridine,
2-methyl-8-(2,4,6-trimethylbenzyloxy)-imidazo[1,2-a]pyridine and

8-(2,6-difluorobenzyloxy)-2-methyl-imidazo[1,2-a]pyridine.

Example V

8-Benzylamino-3-cyanomethyl-2-methyl-imidazo[1,2-a]pyridine

*Step A:* Preparation of 2-amino-3-benzylamino-pyridine.

A mixture of 2,3-diaminopyridine (45 g), ethanol (500 ml), triethylamine (42 g) and benzylbromide (70.5 g) was stirred at room temperature for 3 days. The solvent was evaporated in vacuo and the residue partitioned between chloroform (500 ml) and water (400 ml). The organic layer was reparated, filtered through a silica gel plug and evaporated in vacuo. The residue was triturated with hot chlorobutane to yield a brown powder.

*Step B:* Preparation of title compound.

2-Amino-3-benzylamino-pyridine (17.6 g) obtained according to Step A, 3-chloro-4-oxopentanonitrile (11.6 g) and methanol (500 ml) were stirred together at room temperature for about 1 day. The methanol was then evaporated in vacuo and the residue partitioned between 1N NaOH (350 ml) and ethylacetate (500 ml) and the organic layer was separated, dried over $Na_2CO_3$ and evaporated. Chromatography on silica gel using $CHCl_3$ yielded a brown oil which was dissolved in ether (700 ml). Etheral HCl was added and the HCl-salt of the desired product precipitated. Recrystallization from methanol gave the pure HCl salt which was transformed into desired product (m.p. 204—205°C).

Example VI

8-Benzyloxy-2-methoxycarbonyl-3-methyl-imidazo[1,2-a]pyridine

To a cloudy yellow solution of 2-amino-3-benzyloxy-pyridine (20 g) in 200 ml DME (dimethylethane) there was added a solution of methyl 3-bromo-2-oxo-butyrate (19.5 g) in 100 ml DME. The flask was flushed with nitrogen, closed and left to stir at room temperature for 43 hrs. The reaction mixture was filtered and the solid washed with ether (200 ml) leaving a light tan powder (m.p. 150°C). 34 g of this intermediate was dissolved in 300 ml methanol, stirred and there was added a molecular sieve (type 3A) (25 g) in form of pellets. The mixture was heated under reflux and $N_2$ for 20 hrs. The sieve (now fine powder) was filtered off and the filtrate evaporated. The solid was treated with ethylacetate and this mixture was concentrated almost to dryness. 300 ml of ether was added and the mixture stirred for a few minutes. The solid was filtered off, washed with ether. The product which is obtained as the HBr-salt, had a m.p. of 147.5—149°C (dc).

The free base was obtained by partitioning the HBr between chloroform, ether and 10% aqueous $NaHCO_3$. A slightly grey powder was obtained which was triturated with ether (100 ml) filtered and recrystallized from ethylacetate to give the pure product (m.p. 108.5—109.5°C).

Example VII

8-(2-Fluorobenzyloxy)-3-hydroxymethyl-2-methyl-imidazo[1,2-a]pyridine

*Step A:* Preparation of 8-(2-Fluorobenzyloxy)-2-methyl-imidazo[1,2-a]pyridine-3-carboxylic acid ethyl ester

A mixture of 25.0 g of 2-amino-3-(2-fluorobenzyloxy)-pyridine, 20.5 g ethyl 2-chloroacetoacetate, 10.0 g of sodium bicarbonate and 250 ml dimethoxyethane was heated under reflux for 48 hrs. An additional 5.0 g ethyl 2-chloroacetoacetate was added and refluxed for another 24 hrs., followed by another 5.0 g and refluxed for a further 66 hrs. The mixture was cooled and suction-filtered. The solids collected were washed with tetrahydrofuran, and the combined filtrate and washings were evaporated *in vacuo* to leave a dark-colored oil. Hexane (200 ml) was added to the oil with vigorous stirring, and the resulting precipitate filtered off. To this solid was added (with vigorous stirring) acetonitrile (50 ml) and diisopropyl ether (200 ml) and stirring was continued for 0.5 hr. The suspended solid was filtered and crystallized from acetonitrile to give the desired ester.

Substitution of 2-amino-3-(4-methoxybenzyloxy)-pyridine, 2-amino-3-(2-phenylethoxy)-pyridine, 2-amino-3-(4-fluorobenzyloxy)-pyridine, 2-amino-3-(4-chlorobenzyloxy)-pyridine, 2-amino-3-(4-trifluoromethylbenzyloxy)-pyridine, 2-amino-3-(2-pyridyl-methoxy)-pyridine, and 2-amino-3-benzyloxy-pyridine for the 2-amino-3-(2-fluorobenzyloxy)-pyridine and by substantially following the same procedure, the ethyl esters of the following compounds are produced:

8-(4-chlorobenzyloxy)-2-methyl-imidazo[1,2-a]pyridine-3-carboxylic acid,
8-(4-fluorobenzyloxy)-2-methyl-imidazo[1,2-a]pyridine-3-carboxylic acid,
8-(4-methoxybenzyloxy)-2-methyl-imidazo[1,2-a]pyridine-3-carboxylic acid,
2-methyl-8-(2-phenylethoxy)-imidazo[1,2-a]pyridine-3-carboxylic acid,
2-methyl-8-(4-trifluoromethylbenzyloxy)-imidazo[1,2-a]pyridine-3-carboxylic acid,
2-methyl-8-(2-pyridyl-methoxy)-imidazo[1,2-a]pyridine-3-carboxylic acid,
2-methyl-8-benzyloxy-imidazo[1,2-a]pyridine-3-carboxylic acid.

*Step B:* Preparation of title compound

To an ice-cooled suspension of 1.56 g of lithium aluminium hydride in 200 ml tetrahydrofuran (THF) there was added 15.0 g of 8-(fluorobenzyloxy)-2-methyl-imidazo[1,2-a]pyridine-3-carboxylic acid

ethyl ester in portions so that the temperature remained below 10°C. After stirring at 0—5°C for an additional 1 hr., 1.6 ml water was added dropwise at 0—10°C, followed by 1.6 ml 15% aqueous sodium hydroxide and then 4.8 ml water. The mixture was allowed to warm to room temperature with stirring, and was then suction-filtered in a sintered glass funnel. The solids left in the funnel were thoroughly washed with 200 ml hot tetrahydrofuran and 4 x 150 ml hot chloroform. The filtrate and washings were combined, evaporated *in vacuo*, and the solid residue crystallized from acetonitrile to give the desired compound (m.p. 151—153°C).

By using the appropriate imidazo[1,2-a]pyridine-3-carboxylic acid ethyl esters and by substantially following the procedure of Step B, the following compounds are obtained:

8-(4-chlorobenzyloxy)-3-hydroxymethyl-2-methyl-imidazo[1,2-a]pyridine,
8-(4-fluorobenzyloxy)-3-hydroxymethyl-2-methyl-imidazo[1,2-a]pyridine,
3-hydroxy-8-(4-methoxybenzyloxy)-2-methyl-imidazo[1,2-a]pyridine,
3-hydroxymethyl-2-methyl-8-(2-phenylethoxy)-imidazo[1,2-a]pyridine,
8-benzyloxy-3-hydroxymethyl-2-methyl-imidazo[1,2-a]pyridine,
3-hydroxymethyl-2-methyl-8-(4-trifluoromethylbenzyloxy)-imidazo[1,2-a]pyridine,
3-hydroxymethyl-2-methyl-8-(2-pyridyl-methoxy)-imidazo[1,2-a]pyridine and
8-benzyloxy-3-hydroxymethyl-2-trifluoromethyl-imidazo[1,2-a]pyridine.

Example VIII

3-Cyanomethyl-2-methyl-8-(phenylpropoxy)-imidazo[1,2-a]pyridine

A mixture of 5.3 g of 2-amino-3-(3-phenylpropoxy)-pyridine, 3.2 g of 3-chloro-4-oxopentanonitrile, 2.0 g sodium bicarbonate, and 50 ml dimethoxyethane was heated at reflux for 24 hrs., then stirred at room temperature 66 hrs. the solvent was removed *in vacuo*, the brown residue was taken up in 100 ml methylchloride, filtered, dried over potassium carbonate and evaporated *in vacuo* to leave a brown gum residue. The residue was chromatographed on silica gel using first chloroform, then switching to 1:1 chloroform/ethyl acetate. The early eluates were combined and evaporated and the resultant residue crystallized from isopropyl/ether to give the title compound (m.p. 143—145°C).

Example IX

8-Benzyloxy-2-hydroxymethyl-imidazo[1,2-a]pyridine

To an ice-cooled suspension of 1.5 g lithium aluminium hydride in 150 ml tetrahydrofuran there was added 15.0 g 8-benzyloxy-imidazo[1,2-a]pyridine-2-carboxylic acid ethyl ester (obtained according to Example II) in portions so that the temperature remains below 10°C. After stirring at 0—5°C for an additional 1 hr., 1.5 ml water was added dropwise at 0—10°C, followed by 1.5 ml 15% aqueous sodium hydroxide and then 4.5 ml water. The mixture was allowed to warm to room temperature with stirring, and was then suction-filtered in a sintered glass funnel. The solids left in the funnel were thoroughly washed with 4 x 200 ml hot chloroform.

The filtrate and washings were combined and evaporated *in vacuo* to give the title compound (m.p. 138—140°C).

In a similar manner, reduction of the esters obtained according to Example II leads to the following compounds:

2-hydroxymethyl-8-(4-methoxybenzyloxy)-imidazo[1,2-a]pyridine,
8-(4-fluorobenzyloxy)-2-hydroxymethyl-imidazo[1,2-a]pyridine
2-hydroxymethyl-8-(3-trifluoromethylbenzyloxy)-imidazo[1,2-a]pyridine,
8-(3,4-dichlorobenzyloxy)-2-hydroxymethyl-imidazo[1,2-a]pyridine and
8-(2-fluorobenzyloxy)-2-hydroxymethyl-imidazo[1,2-a]pyridine.

Example X

8-Benzyloxy-2-hydroxymethyl-3-methyl-imidazo[1,2-a]pyridine

To an ice-cooled suspension of $LiAlH_4$ (1.03 g) in tetrahydrofuran (THF) (200 ml) there was added 8-benzyloxy-2-methoxycarbonyl-3-methyl-imidazo[1,2-a]pyridine (13.3 g) obtained according to Example VI in several portions over 20 minutes. The temperature was maintained below +8°C. After all the substance had been added, stirring under cooling was continued for 30 min. and thereafter the mixture was stirred at room temperature for another hour. A further 6 mg of $LiAlH_4$ was added and stirring continued for 1 hr. The reaction mixture was cooled to 3°C and to the stirred mixture there was first added 1.09 ml of water, then 1.63 ml of 10% aqueous NaOH and finally 3.27 ml water. The resulting mixture was stirred for 5 min. on the ice bath and 5 min. at room temperature and then filtered. The solid was washed with 2 x 100 ml of hot THF and wash fluid was added to the original filtrate. The solution was stripped to yield a brown powder which was triturated for 1 hr. in 150 ml $CHCl_3$. After filtration and washing a slightly impure product was obtained (m.p. 172—175°C).

Example XI

8-Benzyloxy-2-chloromethyl-3-imidazo[1,2-a]pyridine

The product of Example IX (9.35 g) was suspended in 50 ml $CHCl_3$. The stirred suspension was cooled and a solution of thionylchloride (8.3 g) in 5 ml $CHCl_3$ was added over a period of 5 min. so as to

keep the temperature below 20°C. A light grey slurry was obtained which was quenched by pouring it into a mixture of aqueous $NaHCO_3$ (25 ml of 1.1M), ice and $CHCl_3$. Water, aqueous $NaHCO_3$ and $CHCl_3$ was added and the resulting mixture having ~450 ml organic phase and 500 ml aqueous phase was stirred until all solid was dissolved. The phases were separated and the aqueous phase extracted with $CHCl_3$. The combined organic phases were washed with brine (2 x 300 ml), dried over $Na_2SO_4$ and stripped to give a residue which was triturated in 100 ml of ether. Filtration and washing of the solid gave the desired product (m.p. 163°C).

Example XII

8-Benzyloxy-3-dimethylaminomethyl-2-methyl-imidazo[1,2-a]pyridine

Into a one-liter flask there was placed 8-benzyloxy-2-methyl-imidazo[1,2-a]pyridine (114 g) (from Example IB), dimethylamine hydrochloride, paraformaldehyde (15.23 g) and methanol (450 ml) and the mixture was refluxed with stirring for 1.5 hrs. Thereafter the mixture was boiled, open to the air, for 3/4 hrs. After cooling to room temperature and treatment with concentrated hydrochloric acid (45 ml) the mixture was stirred for 18 hrs., filtered and the thick white solid mass formed was washed with methanol and 200 ml of anhydrous ether and dried. (M.p. of the hydrochloride salt of the desired compound: 210—211°C).

By reacting the appropriate imidazo[1,2-a]pyridine compounds with dimethylamine hydrochloride and paraformaldehyde in a manner similar to the one described above, the following compounds are obtained:

3-dimethylaminomethyl-8-(4-methoxy-benzyloxy)-2-methyl-imidazo[1,2-a]pyridine,
3-dimethylaminomethyl-2-methyl-8-(3-trifluoromethyl-benzyloxy)-imidazo[1,2-a]pyridine,
3-dimethylaminomethyl-8-(4-fluorobenzyloxy)-2-methyl-imidazo[1,2-a]pyridine,
8-(3,4-dichlorobenzyloxy)-3-dimethylaminomethyl-2-methyl-imidazo[1,2-a]pyridine,
3-dimethylaminomethyl-2-methyl-8-(2-phenylethoxy)-imidazo[1,2-a]pyridine,
8-Benzyloxy-3-dimethylaminomethyl-imidazo[1,2-a]pyridine and the corresponding 2-hydroxymethyl analogues thereof (e.g. 8-benzyloxy-3-dimethylaminomethyl-2-hydroxymethyl-imidazo[1,2-a]pyridine).

Example XIII

8-Benxyloxy-3-cyanomethyl-2-methyl-imidazo[1,2-a]pyridine

*Step A:* Preparation of 8-benzyloxy-3-dimethylamino-methyl-2-methyl-imidazo[1,2-a]pyridine methiodide.

8-Benzyloxy-3-dimethylaminomethyl-2-methyl-imidazo[1,2-a]pyridine hydrochloride (1.325 g) from Example XII, was dissolved in 4.5 liters of hot water. The solution was made strongly basic with 50% NaOH-solution. The chilled mixture was extracted with dichloromethane (3 x 1.5 liters) and the combined extracts were washed with brine (1.5 liters). The dichloromethane extract was concentrated on a rotary evaporator. The residual oil was dissolved in 2.5 liters of ethanol. With stirring the solution was cooled and methyliodide (232 ml) was added dropwise over a period of 1.5 hrs. The mixture was allowed to warm to room temperature overnight under continued stirring. The white precipitate was collected after about 18 hrs. of stirring, washed with 1.5 liters of ethanol and 3 liters of ether. The resulting product is ready for use in Step B below.

By reacting the 3-dimethylaminomethyl-imidazo[1,2-a]pyridines obtained in Example XII with methyliodide according to the process described above, the following quaternary salts are obtained:

3-dimethylaminomethyl-8-(4-methoxy-benzyloxy)-2-methyl-imidazo[1,2-a]pyridine methiodide,
3-dimethylaminomethyl-2-methyl-8-(3-trifluoromethyl-benzyloxy)-imidazo[1,2-a]pyridine methiodide,
3-dimethylaminomethyl-8-(4-fluroobenzyloxy)-2-methyl-imidazo[1,2-a]pyridine methiodide,
8-(3,4-dichlorobenzyloxy)-3-dimethylaminomethyl-2-methyl-imidazo[1,2-a]pyridine methiodide,
3-dimethylaminomethyl-2-methyl-8-(2-phenylethoxy)-imidazo[1,2-a]pyridine methiodide,
8-Benzyloxy-3-dimethylaminomethyl-imidazo[1,2-a]pyridine methiodide and
8-benzyloxy-3-dimethylaminomethyl-2-hydroxymethyl-imidazo[1,2-a]pyridine methiodide.

*Step B:* Preparation of title compound

A mixture of the methiodide of Step A (1.552 g) and sodium cyanide (310 g) in 8.4 liters of dimethylformamide was stirred and heated at a steam bath for 1 hr. The dark brown reaction mixture was poured into 30 liters of ice water and the mixture was stirred for 1 hr. The brown solid product was collected, washed with cold water and allowed to air dry. This material was dissolved in 3.8 liters of hot methanol and treated with hydrogen chloride gas until strongly acidic. The mixture was cooled and the product collected. After washing with methanol, acetonitrile and finally with ether the title product as the hydrochloride salt was obtained.

The salt was resuspended in water and made strongly alkaline with 10% sodium hydroxide. The product was extracted with dichloromethane (3 x 2.5 liters) and the combined extracts concentrated on a rotary evaporator. The residue was dissolved in 3.6 liters of hot acetonitrile, and the resulting solution filtered through a glass wool plug and the filtrate was refrigerated overnight. The desired product was then washed with cold acetonitrile (m.p. 163—166°C).

Using this procedure with the quaternary salts listed above under Example XIII A, thereby applying an apporpriate solvent such as dimethylformamide, dimethylsulfoxide, ethanol, etc., the following compounds are obtained:

3-cyanomethyl-8-(4-methoxybenzyloxy)-2-methyl-imidazo[1,2-a]pyridine,
3-cyanomethyl-2-methyl-8-(3-trifluoromethyl-benzyloxy)-imidazo[1,2-a]pyridine,
3-cyanomethyl-8-(4-fluorobenzyloxy)-2-methyl-imidazo[1,2-a]pyridine,
3-cyanomethyl-8-(3,4-dichlorobenzyloxy-2-methyl-8-(2-phenylethoxy)-imidazo[1,2-a]pyridine,
8-benzyloxy-3-cyanomethyl-2-hydroxymethyl-imidazo[1,2-a]pyridine and,
8-benzyloxy-3-cyanomethyl-imidazo[1,2-a]pyridine.

Example XIV

3-Chloromethyl-8-(2-fluorobenzyloxy)-2-methyl-imidazo[1,2-a]pyridine

A mixture of 8-(2-fouorobenzyloxy)-3-hydroxymethyl-2-methyl-imidazo[1,2-a]pyridine from Example VII (1 g) and phosphorylchloride (15 ml) was heated under reflux for 1 hr. Excess phosphorylchloride was distilled off in vacuo, the residue dissolved in hot ethanolic HCL and the mixture filtered to give the title compound as the hydrochloride salt.

By subjecting other 3-hydroxy-imidazo[1,2-a]pyridines obtained according to Example VII to substantially the same reaction as described above, the hydrochloride salts of the following 3-chloromethyl-compounds are obtained:

8-(4-chlorobenzyloxy)-3-chloromethyl-2-methyl-imidazo[1,2-a]pyridine,
3-chloromethyl-8-(4-fluorobenzyloxy-2-methyl-imidazo[1,2-a]pyridine,
3-chloromethyl-8-(4-methoxybenzyloxy)-2-methyl-imidazo[1,2-a]pyridine,
3-chloromethyl-2-methyl-8-(2-phenylethoxy)-imidazo[1,2-a]pyridine,
8-benzyloxy-3-chlorometyl-2-methyl-imidazo[1,2-a]pyridine,
3-chloromethyl-2-methyl-8-(4-trifluoromethylbenzyloxy)-imidazo[1,2-a]pyridine,
3-chloromethyl-2-methyl-8-(2-pyridyl-methoxy)-imidazo[1,2-a]pyridine and
8-benzyloxy-3-chloromethyl-2-trifluoromethyl-imidazo[1,2-a]pyridine.

Example XV

3-Cyanomethyl-8-(2-fluorobenzyloxy)-2-methyl-imidazo[1,2-a]pyridine

A mixture of 3-chloromethyl-8-(2-fluorobenzyloxy)-2-methyl-imidazo[1,2-a]pyridine hydrochloride from Example XIV (1.3 g), potassium cyanide (1.6 g) and dimethylsulfoxide (20 ml) was stirred at room temperature for 16 hrs.

The mixture was poured into 200 ml cold water and extracted with 2 × 150 ml dichloromethane, the combined organic extracts were dried over potassium carbonate, the mixture filtered, and the filtrate evaporated *in vacuo*. The liquid residue was chromatographed on silica gel (30 g) eluting with ethylacetate-dichloromethane, the fractions containing the product were evaporated *in vacuo*, and the residue was crystallized from acetonitrile to give the desired compound (m.p. 172—173°C). In a similar manner, using the various 3-chloromethyl-imidazo[1,2-a]pyridines obtained in Example XIV the following 3-cyanomethyl-compounds are prepared:

8-(4-chlorobenzyloxy)-3-cyanomethyl-2-methyl-imidazo[1,2-a]pyridine,
3-cyanomethyl-8-(4-fluorobenzyloxy)-2-methyl-imidazo[1,2-a]pyridine,
3-cyanomethyl-8-(4-methoxybenzyloxy)-2-methyl-imidazo[1,2-a]pyridine,
3-cyanomethyl-2-methyl-8-(2-phenylethoxy)-imidazo[1,2-a]pyridine,
8-benzyloxy-3-cyanomethyl-2-methyl-imidazo[1,2-a]pyridine,
3-cyanomethyl-2-methyl-8-(4-trifluoromethyl-benzyloxy)-imidazo[1,2-a]pyridine,
3-cyanomethyl-2-methyl-8-(2-pyridyl-methoxy)-imidazo[1,2-a]pyridine and
8-benzyloxy-3-cyanomethyl-2-trifluoromethyl-imidazo[1,2-a]pyridine.

Example XVI

8-Benzyloxy-2-chloromethyl-imidazo[1,2-a]pyridine

A mixture of 8-benzyloxy-2-hydroxymethyl-imidazo[1,2-a]pyridine (2 g) and phosphorylchloride (10 ml) was heated on a steam bath for 1 hr. Excess phosphorylchloride was distilled off in vacuo, the solid residue triturated with acetonitrile and the mixture filtered to give the title product as the hydrochloride salt.

In substantially the same manner, using the 2-hydroxymethyl-compounds obtained according to Example IX the following 2-chloromethyl-imidazo[1,2-a]pyridine hydrochloride salts are obtained:

2-chloromethyl-8-(4-methoxybenzyloxy)-imidazo[1,2-a]pyridine,
2-chloromethyl-8-(4-fluorobenzyloxy)-imidazo[1,2-a]pyridine,
2-chloromethyl-8-(3-trifluoromethylbenzyloxy)-imidazo[1,2-a]pyridine,
2-chloromethyl-8-(3,4-dichlorobenzyloxy)-imidazo[1,2-a]pyridine,
2-chloromethyl-8-(2-fluorobenzyloxy)-imidazo[1,2-a]pyridine.

Example XVII

8-Benzyloxy-2-cyanomethyl-imidazo[1,2-a]pyridine

A mixture of 1.79 g 8-benzyloxy-2-chloromethyl-imidazo[1,2-a]pyridine, 0.90 g sodium cyanide, and 15 ml dimethylformamide was heated on a steam bath for 1.5 hr. The mixture was cooled and evaporated *in vacuo*, the residue was taken up in chloroform (50 ml) and filtered through a pad of silica gel, the filtrate was evaporated *in vacuo*, and the residue recrystallized from acetonitrile to give the title compound (m.p. 148—150°C).

In a similar manner, reaction of the appropriate chloromethylimidazo[1,2-a]pyridine from Example XVI with an alkali metal cyanide in a suitable solvent leads to the following compounds:

2-cyanomethyl-8-(4-methoxybenzyloxy)-imidazo[1,2-a]pyridine,
2-cyanomethyl-8-(4-fluorobenzyloxy)-imidazo[1,2-a]pyridine,
2-cyanomethyl-8-(3-trifluoromethylbenzyloxy)-imidazo[1,2-a]pyridine,
2-cyanomethyl-8-(3,4-dichlorobenzyloxy)-imidazo[1,2-a]pyridine,
2-cyanomethyl-8-(2-fluorobenzyloxy)-imidazo[1,2-a]pyridine.

Example XVIII

8-Benzyloxy-3-cyanomethyl-imidazo[1,2-a]pyridine-2-methyl-N,N-dimethylglycinate

To an ice-cooled mixture of 8-benzyloxy-3-cyanomethyl-2-hydroxymethyl-imidazo[1,2-a]pyridine (5 g) obtained according to the process of Example XIII, triethylamine (2.6 ml) and tetrahydrofuran (100 ml) there was added, dropwise over 1 hr. a solution of dimethylglycyl-chloride (2.1 g) in tetrahydrofuran (15 ml), and the mixture was stirred an additional 1 hr. as the flask was allowed to warm to room temperature. Solvent was evaporated *in vacuo*, the residue was dissolved in 500 ml chloroform, and washed with 2 x 200 ml cold saturated aqueous sodium bicarbonate. The washed organic extract was evaporated *in vacuo* and the residue was recrystallized from acetonitriletoluene to give the title compound (m.p. 135—137°C).

Example XIX

8-Benzyloxy-2-methyl-imidazo[1,2-a]pyridine-3-(2-butanonitrile)

A mixture of 11.1 g of 8-benzyloxy-3-cyanomethyl-2-methyl-[1,2-a]pyridine, 4.36 g of bromethane, 9.1 g of benzyltriethyl ammonium chloride, 0.2 mol of 50% sodium hydroxide solution, and 200 ml of dichloromethane was stirred for 48 hrs., washed twice with brine, and evaporated *in vacuo*. The residue was dissolved in 50 ml dichloromethane, stirred, and treated slowly with 250 ml of ethyl acetate. After filtration, the solution was treated with charcoal, filtered and evaporated *in vacuo* chromatography on silica gel with methanol/dichloromethane elution and crystallization from isopropyl ether gave the desired product (m.p. 134—136°).

Example XX

8-Benzyloxy-3-(2-hydroxyethyl)-2-methylimidazo[1,2-a]pyridine

A solution of 26 g of 8-benzyloxy-3-cyanomethyl-2-methyl-imidazo[1,2-a]pyridine, 50 ml of 15% sodium hydroxide solution, 100 ml of water, and 100 ml of ethanol was heated at reflux for 6 hrs. The reaction mixture was distilled until 100 ml of distillate had been collected. The aqueous portion was washed twice with dichloromethane and then treated with a 6N hydrochloric acid to pH 6. Filtration afforded 8-benzyloxy-2-methyl-imidazo[1,2-]pyridine-3-acetic acid. 2.0 g of this acid was added to 1 g of lithium aluminium hydride in 100 ml of tetrahydrofuran and was stirred for 5 hrs. at room temperature. The reaction mixture was treated with 1 ml of water, 1 ml of 15% sodium hydroxide solution, then with 3 ml of water. Filtration of salts and evaporation of the filtrate gave the title product after recrystallization from acetonitrile (m.p. 134—138°C).

Example XXI

[8-Benzyloxy-2-methyl-imidazo[1,2a]pyridine-3-yl-methyl]-N,N-dimethylcarbamate

A mixture of sodium hydride (0.26 g) and 8-benzyloxy-3-hydroxymethyl-2-methyl-imidazo[1,2-a]pyridine (2.7 g) in dimethylformamide (15 ml) was heated in a steam bath for 4 min. Dimethylcarbamoyl chloride (1.2 ml) was added and the mixture was stirred for 2.75 hrs. Water (125 ml) was added and the solid product collected and recrystallized from ethanol to give the title compound (m.p. 123—125°C).

Example XXII

8-Benzylthio-3-cyanomethyl-2-methyl-imidazo[1,2-a]pyridine

*Step A:* Preparation of *N*,N-Dimethyl-(2-methylimidazo[1,2-a]pyridine-8-yl) thiocarbamate.

To an ice-cooled solution of 13.0 g 86% potassium hydroxide in 160 ml water there was added 29.6 g 8-hydroxy-2-methyl-imidazo[1,2-a]pyridine, and the mixture was stirred for 0.5 hr. at 0—5°C. To this was added, dropwise over 1 hr., a solution of 33.0 g dimethylthiocarbamoyl chloride in 80 ml tetrahydrofuran. The mixture was allowed to warm to room temperature, stirred an additional 0.25 hr., cooled to 5°C and suction-filtered to leave a yellow solid. This solid was dissolved in chloroform (1 liter), dried over potassium carbonate, treated with charcoal, filtered through a pad of silica gel, and the

filtrate was evaporated *in vacuo*. The viscous brown residue was dissolved in 1.5 liter boiling toluene and the solution was concentrated *in vacuo* to 125 ml and allowed to cool. The deposited crystals were separated by filtration, and recrystallized from toluene to give N,N-dimethyl-(2-methylimidazo[1,2-a]pyridine-8-yl) thiocarbamate.

*Step B:* Preparation of N,N-Dimethyl-(2-methylimidazo[1,2-a]pyridine-8-yl) carbamothioate.

In a 250 ml three-neck flask fitted with a thermometer, a reflux condenser, and a solids-addition adapter containing 13.0 g N,N-dimethyl-(2-methylimidazo[1,2-a]pyridine-8-yl) thiocarbamate, there was placed 150 ml diphenyl ether and the apparatus was flushed with nitrogen. While a slow stream of nitrogen was passed through, the flask was heated. When the diphenyl ether reached 240°C, the N,N-dimethyl-(2-methylimidazo[1,2-a]pyridine-8-yl)thiocarbamate was added all at once from the solids-addition adapter, and the mixture was heated under reflux for 0.75 hr. The mixture was cooled to 50°C, poured slowly into 850 ml hexane with stirring, and then the mixture cooled to 16°C and kept at this temperature for 16 hrs. The mixture was filtered, and the solid crystallized from acetonitrile to give N,N-dimethyl-(2-methylimidazo[1,2-a]pyridine-8-yl)carbamothioate.

*Step C:* Preparation of 8-benzylthio-2-methylimidazo[1,2-a]pyridine.

A mixture of 10.0 g N,N-dimethyl-(2-methylimidazo[1,2-a]pyridine-8-yl)carbamothioate, 3.0 g 85% potassium hydroxide and 250 ml ethanol was under reflux for 24 hrs. while an atmosphere of nitrogen was maintained in the apparatus. The mixture was allowed to cool to room temperature. To the so obtained thiol there was added benzylchloride (6.9 g) and the mixture was stirred at room temperature for 16 hrs. The solvent was evaporated *in vacuo*, the residue was taken up in 300 ml chloroform and washed with 75 ml water. The organic layer was separated and dried over potassium carbonate, filtered, and the filtrate was evaporated *in vacuo*. The residue was recrystallized from acetonitrile to give the desired compound.

*Step D:* Preparation of 8-benzylthio-3-dimethylaminomethyl-2-methyl-imidazo[1,2-a]pyridine.

A mixture of 10.3 g 8-benzylthio-2-methyl-imidazo[1,2-a]pyridine, 2.75 g. dimethylamine hydrochloride, 1.15 g paraformaldehyde and 75 ml ethanol was heated under reflux for 2 hrs. and then stirred 16 hrs. at room temperature. The mixture was evaporated *in vacuo*, and the residue was dissolved in 250 ml water. The solution was made basic with 10% aqueous sodium hydroxide, saturated with sodium chloride and extracted with chloroform (3 x 250 ml). The combined extracts were dried over potassium carbonate, filtered, the filtrate evaporated *in vacuo* and the residue triturated with ether and filtered to give the desired product.

*Step E:* Preparation of 8-benzylthio-3-dimethylaminomethyl-2-methyl-imidazo[1,2-a]pyridine methiodide.

A solution of 3.2 g 8-benzylthio-3-dimethylaminomethyl-2-methyl-imidazo[1,2-a]pyridine, 50 ml ethanol, 50 ml acetone and 0.64 ml iodomethane was stirred at room temperature for 18 hrs. The resulting mixture was evaporated *in vacuo* to leave the desired methiodide.

*Step F:* Preparation of final compound.

A mixture of 4.4 g 8-benzylthio-3-dimethyl-aminomethyl-2-methyl-imidazo[1,2-a]pyridine methiodide, 1.6 g finely powdered sodium cyanide, and 40 ml dimethylformamide was heated on a steam bath for 0.75 hr. and the resulting mixture was poured into 400 ml water. The precipitate was separated by suction filtration, dried, and recrystallized from ethyl acetate-hexane to give 8-benzylthio-3-cyanomethyl-2-methyl-imidazo[1,2-a]pyridine.

Example XXIII

8-Benzylsulfinyl-3-cyanomethyl-2-methylimidazo[1,2-a]pyridine

To an ice cooled, stirred solution of 8-benzylthio-3-cyanomethyl-2-methyl-imidazo[1,2-a]pyridine (4.8 g) in 50 ml dichloromethane there was added in small portions over 0.25 hr., 3.35 g of 85% 3-chloroperoxybenzoic acid, and the mixture was stirred an additional 0.25 hr. at 0°C. The mixture was suction-filtered cold, the filtrate diluted to 200 ml with chloroform, and the solution washed with 100 ml 10% aqueous sodium bisulfite and 2 x 75 ml 5% aqueous sodium bicarbonate. The organic extract was evaporated *in vacuo*, and the residue crystallized from toluene to give the title compound (m.p. 183—185°C).

Example XXIV

8-Benzylsulfonyl-3-cyanomethyl-2-methylimidazo[1,2-a]pyridine

To an ice cooled, stirred solution of 8-benzylsulfinyl-3-cyanomethyl-2-methyl-imidazo[1,2-a]pyridine (4.8 g) in 50 ml dichloromethane there was added in portions over 0.25 hr., 3.35 g of 85% 3-chloroperoxybenzoic acid. The mixture was warmed to room temperture, an additional 3.35 g of 85% 3-chloroperoxybenzoic acid was added in portions over 10 minutes and the resulting mixture was stirred 0.5 hr. at ambient temperature, then heated under reflux 2 minutes. The mixture was diluted to

250 ml with chloroform, washed with 125 ml cold 20% aqueous sodium sulfite and 2 x 200 ml cold 5% aqueous sodium bicarbonate, and then dried over potassium carbonate. The solution was poured into a column of silica gel (50 g) packed in chloroform and eluted with ethyl acetate-chloroform. The fractions containing the product were evaporated *in vacuo*, and the residue crystallized from acetonitrile to give the desired compound (m.p. 200—201°C).

Example XXV

8-Benzyloxy-3-cyanomethyl-2-methylthiomethylimidazo[1,2-a]pyridine

A solution of 25 ml methanthiol in 125 ml 2-propanol was treated with 4.0 g of 8-benzyloxy-2-chlormethyl-3-cyanomethyl-imidazo[1,2-a]pyridine and 4.3 ml of 3N NaOH-solution and the mixture was stirred for three hours. The solvent was removed in vacuo and the resulting residue was recrystallized from ethylacetate/hexane to give the title compound (m.p. 107°C).

Example XXVI

8-Benzyloxy-3-cyanomethyl-2-methylsulfinylmethylimidazo[1,2-a]pyridine

A solution of 1.34 g 8-benzyloxy-3-cyanomethyl-2-methylthiomethyl-imidazo[1,2-a]pyridine was cooled to 0° and treated with 0.9 g *m*-chloroperoxybenzoic acid (85% purity) in small portions over 15 minutes. The solution was diluted with $CHCl_3$; washed successively with 5% aqueous sodium bisulfite, 5% aqueous potassium carbonate, and brine; dried over potassium carbonate; and evaporated *in vacuo.* The residue was chromatographed on silica gel (60 g) eluting with 1:1 methylene chloride/tetrahydrofuran. The fractions containing the desired product were combined and evaporated, and the residue recrystallized from tetrahydrofuran/ethyl ether to give the title compound (m.p. 145—147°C).

Example XXVII

8-Benzyloxy-3-cyanomethyl-2-methylsulfonylmethylimidazo[1,2-a]pyridine

A solution of 1.76 g of 8-benzyloxy-3-cyanomethyl-2-methylthiomethyl-imidazo[1,2-a]pyridine was cooled to 0° and treated with 2.3 g *m*-chloroperoxybenzoic acid (85% purity), and allowed to warm to room temperature while stirring for one-half hour. The mixture was then cooled to 0° and suction-filtered. The filtrate was washed successively with 5% aqueous sodium bisulfite, 5% aqueous potassium carbonate, and brine; dried over potassium carbonate; and evaporated *in vacuo*. The residue was crystallized from acetonitrile/2-propyl ether to give the title compound (m.p. 163—164°C).

Example XXVIII

3-Cyanomethyl-2-methyl-8-(2-thienylmethoxy)imidazo[1,2-a]pyridine

A mixture of 2-amino-3-(2-thienylmethoxy)-pyridine (5.35 g), 3-chloro-4-oxopentanonitrile (4.5 g) and triethylamine was stirred together in ethanol and heated under reflux for 27 hours. The solvent was evaporated in vacuo and the residue was partitioned between $CH_2Cl_2$ (100 ml) and 15% $K_2CO_3$ (30 ml/. The organic layer was separated, washed with 15% $K_2CO_3$ (2 x 30 ml) and dried ($Na_2SO_4$). The solvent was evaporated in vacuo and the residue was recrystallized from acetonitrile to yield the title compound (m.p. 155—157°C).

Example XXIX

8-Benzyloxy-3-cyanomethyl-2-methylimidazo[1,2-a]pyridine

3-cyanomethyl-8-hydroxy-2-methyl-imidazo[1,2-a]pyridine (1.0 g) was dissolved in DMF (25 ml) and sodiumhydride (0.26 g, 50% in o.e.) was added. The mixture was stirred at room temperature for 30 minutes and then benzylchloride (0.7 g) was added and stirring while heating to 60°C was continued for 3 hrs. The solvent was evaporated off in vacuo, the residue partitioned between chloroform and water, the organic layer separated, dried ($MgSO_4$) and evaporated in vacuo. The residue was recrystallized from acetonitrile to give the title compound (m.p. 163—166°C).

Example XXX

8-Benzyloxy-3-cyanomethyl-2-methylimidazo[1,2-a]pyridine

A suspension of 8-benzyloxy-2-methyl-imidazo[1,2-a]pyridine-3-acetamide (3 g) in dixane was cooled to 5—10°C. To the suspension there was added over a period of 5 minutes a solution of trifluoroacetic anhydride (6 g) in dixane. The mixture was maintained cool and stirred for 30 minutes. Thereafter the mixture was allowed to warm to room temperature and was stirred for a further four hrs. The mixture was then diluted with ice water and the precipitate was filtered off. After recrystallization from ethylacetate the desired compound was obtained (m.p. 163—166°C).

Example XXXI

8-Benzyloxy-2-methyl-5,6,7,8-tetrahydroimidazo[1,2-a]pyridine

*Step A:* Preparation of 8-hydroxy-2-methyl-5,6,7,8-tetrahydroimidazo[1,2-a]pyridine hydrochloride.

A mixture of 8-benzyloxy-2-methyl-imidazo[1,2-a]pyridine (5.9 g), 6.1 N HCl (4.2 ml), 10% Palladium-on-charcoal (5.9g) and 240 ml ethanol was hydrogenated under a $H_2$-pressure of about

3.5 kg/cm$^2$ for 22 hrs. at room temperature, after which period a further 2g of fresh catalyst was added and hydrogenation continued for another 45 hours. After filtration and evaporation of the solvent at the reduced pressure, the title compound is obtained as a pale yellow solid with m.p. 140—152°C.

*Step B:* Preparation of 8-benzyloxy-2-methyl-5,6,7,8-tetrahydroimidazo[1,2-a]pyridine.

The compound of Step A (3.49 g) was suspended in N,N-dimethylformamide (25 ml) and potassium t-butoxide (2.18 g) was added. The mixture was stirred 5 min. at ice bath temperatures and 10 min. at room temperature and thereafter the mixture was cooled to ice bath temperature again.

This reaction mixture (which contains the free base of the compound of Step A) was added stepwise to an ice cooled, stirred suspension of 1.02 g of a 50% dispersion of sodium hydride. The resulting mixture is stirred 30 min. on an ice-water bath. To this cold suspension (which contains the alkoxide of the compound of Step A) there was added a solution of benzylchloride (2.48 g) in DMF. The resultant mixture was stirred in an ice bath for 5 min., at room temperature for 5 min. and then in a heating bath at 85—90°C for 1.5 hrs. The solvent was evaporated off at reduced pressure. To the residue there was added water and the mixture was extracted three times with a 1:1 mixture of methylenchloride and ether. The combined extracts were washed with brine, dried over $Na_2SO_4$ and the solvent evaporated at reduced pressure. The oily residue was chromatographed on silica gel, using a 1:1 mixture of acetaone and methylene chloride as eluant. The title compound was obtained as a yellow oil with the following PMR spectrum (DMSO-$d_6$): $\delta$ 2.08 (3H,s); 1.7—2.2 (4H,m); 3.65—4.1 (2H,m); 4.42 (Ih, deformed triplet); 4.70 (2H,s); 6.73 (IH,s); 7.30(SH,s).

Example XXXII

8-Benzyloxy-3-dimethylaminomethyl-2-methyl-5,6,7,8-tetrahydroimidazo[1,2-a]pyridine

A mixture consisting of the product of Example XXXII (1.52 g), dimethylamine hydrochloride (565 mg), paraformaldehyde (238 mg) and ethanol (10 ml) was refluxed for four hrs. After cooling to room temperature, the solvent was evaporated under reduced pressure. The residue was dissolved in chloroform, the solution was washed four times with 5% aqueous potassium carbonate, dried over $Na_2SO_4$ and then the solvent was evaporated off on a rotary evaporator. The residue was chromatographed on silica gel, eluting with acetone, to obtain the title compound (m.p. 68—70°C).

Example XXXIII

8-Benzyloxy-3-cyanomethyl-2-methyl-5,6,7,8-tetrahydroimidazo[1,2-a]pyridine

*Step A:* Preparation of 8-benzyloxy-3-dimethylaminomethyl-2-methyl-5,6,7,8-tetrahydroimidazo[1,2-a]pyridine methiodide.

To a solution of 8-benzyloxy-3-dimethylaminomethyl-2-methyl-5,6,7,8-tetrahydroimidazo[1,2-a]pyridine (2.56 g) in acetone (35 ml) there was added a solution of methyliodide (1.22 g) in acetone (2 ml). The resulting solution was stirred for 16 hrs. at room temperature. The solvent was then evaporated on a rotary evaporator and the last traces (of solvent) were removed under high vacuum. The so-obtained title compound was a hydroscopic powder with a very broad melting range having the following PMR spectrum (DMSO-$d_6$): $\delta$-2.0 (4H,m); 2.25 (3H,s); 3.05 (pH,s); 3.8—4.2 (2H,m); 4.55 (2H, broad singlet)

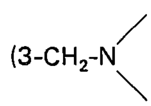 and $H_8$);

4.75 (2H,s); 7.30 (5H, broad singlet).

*Step B:* Preparation of title compound.

The compound of Step A (3.3 g) and potassium cyanide (1.22 g) were added to dimethylformamide (30 ml) and the mixture was stirred at 90°C for 90 min. The solvent was removed under reduced pressure and the residue was dissolved in a mixture of chloroform and water. The layers were separated and the aqueous phase was extracted three times with chloroform. The chloroform fractions were combined, washed with brine, dried over $Na_2SO_4$ and evaporated on a rotary evaporator. The residue was chromatographed on silica gel, eluting with a 1:1 mixture of acetone and methylene chloride, and the title compound is obtained (m.p. 131.5—134.5°C).

Example XXXIV

3-Cyanomethyl-2-methyl-8-phenoxyimidazo[1,2-a]pyridine

A mixture of 8-hydroxy-2-methyl-imidazo[1,2-a]pyridine (14.8 g) and bromobenzene (15.7 g) and copper oxide (1.0 g) in dimethylacetamide was heated under reflux for 48 hrs. The solvent was removed by distillation and the residue suspended in dichloromethane, washed with 10% sodium hydroxide solution and the dichloromethane was evaporated to give 2-methyl-8-phenoxy-imidazo[1,2-a]pyridine. Treatment of this compound in accordance with Examples XII and XIII yields the desired product.

Example XXXV

8-Benzyloxy-2-methyl-imidazo[1,2-a]pyridine-3-(3-propanonitrile)

A solution of 3-(8-benzyloxy-2-methyl-imidazo[1,2-a]pyridylmethyl)-t-butylcyanoacetate (8 g) and p-toluenesulfonic acid hydrate (0.5 g) in dichloromethane (30 ml) was evaporated in vacuo. The residue was heated in an oil bath, held at 145°—150°C for 5 1/2 hrs., dissolved in dichloromethane (100 ml) and washed with 5% sodium hydroxide solution. Evaporation of the dichloromethane an chromatography on silica gel of the residue (elution with methanol/dichloromethane) gave the desired product which after recrystallization from ethylacetate had a m.p. of 130°—131°C.

Example XXXVI

8-Benzyloxy-2-methyl-imidazo[1,2-a]pyridine-3-acetamide

A mixture of 8-benzyloxy-3-(2-cyanomethyl)-2-methyl-imidazo[1,2-a]pyridine (6 g), 15% NaOH (10 ml), $H_2O$ (50 ml) and methanol (30 ml) was refluxed for 3 hrs. The methanol was removed in vacuum and the solid precipitate was filtered and washed with water. Recrystallization from methanol gave the desired amide (m.p. 219—221°C).

Example XXXVII

8-Benzyloxy-2-methyl-imidazo[1,2-a]pyridine-3-thioacetamide

A stirred mixture of 8-benzyloxy-3-cyanomethyl-2-methyl-imidazo[1,2-a]pyridine (1 g) and dry pyridine (2 ml) was kept under nitrogen and cooled to −20°C. $H_2S$ was bubbled in for 10 min. and triethylamine (0.5 ml) was added and the reaction flask was sealed off. The mixture was allowed to warm to room temperature and was stirred overnight. Then the mixture was poured into 25 ml of cold water while stirring and the precipitate formed was suction-filtered and dried. The solid was dissolved in DMF (3 ml) on a steam bath, and $H_2O$ (3 ml) was added. After cooling the precipitate is filtered off (m.p. 190°—192°C).

As is apparent to anyone skilled in the art, one may, by following the appropriate process of those exemplified above, prepare all further compounds falling within the scope of this invention, such as those defined in the Table below.

TABLE

| $R_2$ | $R_3$ | X | Z–T–Ar |
|---|---|---|---|
| $-CH_3$ | $-CH_2-O-CO-CH_3$ | H | benzyloxy |
| $-CO-O-C_2H_5$ | $-CH_2-N(CH_3)_2$ | H | benzyloxy |
| $-CH_3$ | $-CH_2-N(CH_3)_2$ | H | 2-pyridyl-methoxy |
| $-CH_3$ | $-CH(OH)-CH_3$ | H | benzyloxy |
| $-CH_3$ | $-CH_2OH$ | H | 3,4-dichlorobenzyloxy |
| $-CH_3$ | $-CH_2OH$ | H | 4-t-butylbenzyloxy |
| $-CH_3$ | $-CH_2OH$ | H | 3-trifluoromethylbenzyloxy |
| $-CH_3$ | $-C(CH_3)_2-CN$ | H | benzyloxy |
| $-CH_3$ | $-CH_2-O-CO-C(OH_3)_3$ | H | benzyloxy |
| $-CH_3$ | H | H | benzylthio |
| $-CH_3$ | $-CH_2CN$ | H | 4-pyridyl-methoxy |
| $-CH_3$ | H | H | 2-phenylethoxy |
| $-CH_3$ | $-CH_3$ | H | benzyloxy |
| $-CH_3$ | H | H | 2-phenylethyl |
| $-CH_3$ | $-CH_2-N(CH_3)_2$ | H | benzylthio |
| $-CH_3$ | $-CH(CH_3)-CN$ | H | benzyloxy |
| $-CH_3$ | $-CH_2CN$ | H | 4-t-butylbenzyloxy |
| $-C(CH_3)_3$ | H | H | benzyloxy |
| $-CH_3$ | $-CH_2-N(CH_3)_2$ | H | 4-trifluoromethylbenzyloxy |
| $-CH_3$ | H | H | 4-t-butylbenzyloxy |
| $-CH_3$ | $-CH_2CN$ | H | 1-phenylethoxy |
| $-CH_3$ | $-CH_2CN$ | H | phenylethyl |
| $-CH_3$ | $-CH_2CN$ | $-(5)CH_3$ | benzyloxy |
| $-CH_3$ | H | H | 2,4,6-trimethylbenzyloxy |

TABLE (Continued)

| $R_2$ | $R_3$ | X | Z–T–Ar |
|---|---|---|---|
| $-CH_3$ | $-CH_2N(CH_3)_2$ | H | 2,4,6-trimethylbenzyloxy |
| $-CH_3$ | $-CH_2OH$ | H | phenylethyl |
| $-CH_3$ | $-CH_2CN$ | H | 2,4,6-trimethylbenzyloxy |
| $-CH_3$ | $-CH_2CH_3$ | H | benzyloxy |
| $-CH_2-O-CO-C(CH_3)_3$ | $-CH_2CN$ | H | benzyloxy |
| $-CH_2OH$ | H | H | benzyloxy |
| $-CH_3$ | H | H | 2-pyridyl-methoxy |
| $-CH_3$ | $CH_2CN$ | H | 3-pyridyl-methoxy |
| $-CH(CH_3)_2$ | H | H | benzyloxy |
| $-CH_3$ | $CH_2CN$ | H | 4-methylsulfonylbenzyloxy |
| $-CH_3$ | $-CH_2CN$ | –(6)Cl | benzyloxy |
| $-CH_2-CH_3$ | H | H | benzyloxy |
| $-CH_2-CH_3$ | $CH_2CN$ | H | benzyloxy |
| 2-pyridyl | H | H | benzyloxy |
| phenyl | H | H | benzyloxy |
| p-methylsulfonyl-phenyl | H | H | benzyloxy |
| p-methylsulfonyl-phenyl | $CH_2CN$ | H | benzyloxy |
| $-CH_2Br$ | H | H | benzyloxy |
| $-CH_3$ | $-CH_2-N(CH_3)_2$ | H | 4-cyanobenzyloxy |
| $-CH_3$ | H | H | 4-cyanobenzyloxy |
| $-CH_3$ | $CH_2CN$ | H | 4-cyanobenzyloxy |
| $-CH_3$ | $-CH_2-CS-NH_2$ | H | benzyloxy |
| $-CH_3$ | $-CH_2-CO-NH_2$ | H | benzyloxy |
| $-CH_3$ | $-CH_2-O-C_2H_5$ | H | benzyloxy |

TABLE (Continued)

| $R_2$ | $R_3$ | X | Z–T–Ar |
|---|---|---|---|
| $-CH_3$ | $-CH(CH_3)-O-CH_3$ | H | benzyloxy |
| $-CH_3$ | $-CH_2-CO-N(CH_3)_2$ | H | benzyloxy |
| $-CH_3$ | N,N-pentamethylene-carboxymethyl | H | benzyloxy |
| $-CH_3$ | $-CH_2-O-CH_3$ | H | benzyloxy |
| $-CH_3$ | 3-morpholino-carboxymethyl | H | benzyloxy |
| $-CH_3$ | phenyloxymethyl | H | benzyloxy |
| $-CH_3$ | $-CH_2-CS-N(CH_3)_2$ | H | benzyloxy |
| $-CH_3$ | $-CH_2-CN$ | H | phenylpropyloxy |
| $-CH_3$ | $-CH_3$ | H | benzylamino |
| $-CH_3$ | $-CH_2CN$ | H | 3-thienyl-methoxy |

As is also obvious to anyone skilled in the art, the respective X substituted analogues of the (X-unsubstituted) compounds in the above Table and in the Examples may also be obtained by the processes above by using the appropriate X-substituted starting compounds.

The compounds of this invention (1) have the applied use characteristics of being capable of inhibiting gastric acid secretion *and* of exerting a cytoprotective effect (sometimes also referred to in the art as mucoprotective effect) which characteristics enable the compounds to relieve the symptoms of peptic ulcer disease (including stress ulceration) and promote healing of gastric and/or duodenal ulcers. In addition to the antisecretory effects of the compounds of this invention, observations suggest a cytoprotective action which may enhance its projected anti-ulcer activity. Indications of this include a significant inhibition of absolute alcohol-induced gastric necrosis following administration of the compounds of this invention. Thus the compounds of this invention serve as inhibitors of gastric ulceration (or other damage to the gastro-intestinal tract) due to aspirin (and other damage-causing anti-inflammatory/analgesic compounds) and thus the compounds (I) are useful as conjunctive therapeutic agents for coadministration with such anti-inflammatory/analgesic agents such as aspirin, indomethacin, phenylbutazones, ibuprofen, naproxen, tolmetin and other agents having the untoward side effect of contributing to damage to the gastro-intestinal tract.

The compounds of this invention, in order to be evaluated for their applied use characteristics undergo testing procedures according to standard biological procedures wherein the compounds are evaluated both on absolute basis and on a comparative basis with compounds known to possess the characteristics useful for the treatment and/or prevention of peptic ulcer disease, duodenal ulcer disease and drug induced gastric ulceration. Such tests include testing in dogs prepared under aseptic surgical conditions with either Heidenhain gastric pouches or simple gastric fistulas fitted to facilitate collection of gastric secretions. The test compounds are administered in appropriate and well-defined and well-known vehicles for either intravenous delivery or oral administration. The agonist employed to stimulate gastric secretion include such compounds as histamine, pentagastrin, and feeding in dogs equipped with Heidenhain pouches, and insulin hypoglycemia (in addition to histamine and pentagastrin) in dogs with gastric fistulas.

Caesarean-derived Sprague-Dawley male rats are used for gastric secretion with pyloric ligation techniques and anti-ulcer studies employing aspirin-induced ulceration.

From these and other tests, as well as by comparison with known anti-secretory (anti-ulcer

agents) the compounds of this invention are found to be effective for the oral treatment of the ulcerative disease states herein mentioned at doses of about 0.5 to 50 mg per kilogram of body weight per day, preferably being administered in 3—4 divided doses per day. In those instances wherein it is desired to administer the compounds of this invention via a parenteral route (e.g. intravenously) the compounds (I) are administered at a dose range of about 0.01 to 10 mg/kg in single or multiple daily doses. Of course, the dose will be regulated by the attending diagnostician dependind on factors such as the degree and severity of the disease state.

The recommended dose range for the preferred compounds of this invention is the oral dose of 75 to 1600 mg/day in three to four divided doses to achieve relief of the symptoms of peptic ulcer disease and promote the healing of gastric and/or duodenal ulcers.

In their use in the treatment of peptic ulcer disease, gastric and duodenal ulcers, and in the prevention and treatment of drug-induced gastric ulceration, the compounds are administered in unit dosage forms such as tablets, capsules, pills, powders, granules, sterile parenteral solutions or suspensions, suppositories and the like. Such preparations are prepared according to standard techniques well known in the art. A few examples of such pharmaceutical formulations are as follows:

FORMULATION A
Tablets

| No. | Ingredient | mg/tab | mg/tab |
|---|---|---|---|
| 1 | 8-Benzyloxy-3-cyano-methyl-2-methyl-imidazo [1,2-]pyridine (micronized) | 25.0 | 400.00 |
| 2 | Lactose impalpable powder USP | 114.00 | 241.50 |
| 3 | Corn starch USP | 25.00 | 50.00 |
| 4 | Corn starch as 5% paste in distilled water | 10.00 | 35.00 |
| 5 | Corn starch USP | 25.00 | 50.00 |
| 6 | Magnesium stearate USP | 1.00 | 3.50 |
| | | 200.00 | 700.00 |

Method of Manufacture

Mix item nos. 1, 2 and 3 in a suitable blender 5 to 15 minutes. Pass through a fine screen (#40) if necessary. Reblend for 5 to 10 minutes and granulate with item no. 4. Pass the damp granulated mass through a coarse sieve (#6) using a suitable mill. Dry the damp granules at 40 to 50°C overnight. Mill the dried granules using no. 20 screen. Add item no. 5 and blend for 5 to 10 minutes. Add item no. 6 and blend further for 3 to 5 minutes. Compress the tablet mixture into an appropriate size and weight tablets using suitable tablet machine.

FORMULATION B
Capsules

| No. | Ingredient | mg/tab | mg/tab |
|---|---|---|---|
| 1 | 8-Benzyloxy-3-cyano-methyl-2-methyl-imidazo[1,2-a] pyridine | 25.00 | 400.00 |
| 2 | Lactose, impalpable powder USP | 144.00 | 191.50 |
| 3 | Corn starch USP | 30.00 | 105.00 |
| 4 | Magnesium stearate USP | 1.00 | 3.50 |
| | | 200.00 | 700.00 |

Method of Manufacture

Mix item nos. 1, 2 and 3 in a suitable blender for 5 to 10 minutes. Pass through a fine screen (#40) if necessary. Reblend for 5 to 10 minutes, add item no. 4 and mix further for 3 to 50 minutes. Using a suitable machine, encapsulate the mixture in to a two piece hard gelatin capsule of appropriate size.

FORMULATION C
Suspensions

| Ingredients | Formula A mg/ml | Formula B mg/ml |
|---|---|---|
| 8-Benzyloxy-3-cyanomethyl-2-methyl-imidazo[1,2-a]pyridine | 5.0 | 80.0 |
| Sucrose | 600.0 | 600.0 |
| Benzyl alcohol | 10.0 | 10.0 |
| Methylcellulose (15 cps) | 4.0 | 4.0 |
| Polysorbate 80 | 5.0 | 5.0 |
| Vanillin | 0.2 | 0.2 |
| Purified Water q.s. | 1.0 ml | 1.0 ml |

Method of Manufacture

1. Charge approximately 40% of the final volume of purified water in a stainless steel tank. Heat to boiling. Agitate using an appropriate stirrer. Agitation should continue throughout procedure.
2. Add sucrose until it is dissolved.
3. Slowly add methylcellulose until it is well dispersed.
4. Start cooling the mixture to room temperature.
5. Add polysorbate, benzyl alcohol and vanillin until all ingredients are well dispersed.
6. Add the active ingredient until a uniform dispersion is formed.
7. This suspension is then q.s. to final volume with purified water at 25°C.

FORMULATION D
Parenterals

| INJECTION | mg/ml |
|---|---|
| 8-Benzyloxy-3-cyanomethyl-2-methyl-imidazo[1,2-a]pyridine | 25.00 |
| Methylparaben | 1.30 |
| Propylparaben | 0.20 |
| Sodium bisulfite | 3.20 |
| Disodium edetate | 0.20 |
| Sodium sulfate | 2.60 |
| Water for injections q.s. ad | 1.0 ml |

Method for Manufacture

1. Dissolve parabens in a portion (approximately 85% of the final volume) of the water for injection at 65—70°C.
2. Cool to 25—35°C. Charge and dissolve sodium bisulfite, disodium edetate and sodium sulfate.
3. Charge and dissolve the active ingredient.
4. Bring the solution to the final volume by adding water for injection.
5. Filter the solution through 0.22 u membrane and fill into appropriate containers.
6. Terminally sterilize the units by autoclaving.

FORMULATION E
Injectable Suspension

| Injectable Suspension | mg/ml |
|---|---|
| 8-Benzyloxy-3-cyanomethyl-2-methyl-imidazo[1,2-a]pyridine, sterile | 50.0 |
| Benzyl alcohol | 9.0 |
| Methylparaben | 1.8 |
| Propylparaben | 0.2 |
| Sodium carboxymethylcellulose | 5.0 |
| Polyethylene Glycol 4000 | 10.0 |
| Povidone | 5.0 |
| Sodium Citrate | 15.0 |
| Disodium edetate | 0.1 |
| Water for injection | q.s |
| To make | 1.0 ml |

Method of Preparation

1. Dissolve parabens in a portion of water for injection at 65—70°C.
2. Cool to 25—35°C. Charge and dissolve benzyl alcohol, sodium citrate, disodium edetate, PEG 4000, povidone and sodium carboxymethylcellulose.
3. Filter the solution and sterilize by autoclaving.
4. Make a slurry of the sterile active ingredient and pass it through a colloid mill.
5. Mix it well with solution from Step 3 and pass it through the mill.
6. Bring the suspension to the final volume/weight.

FORMULATION F SUPPOSITORIES

imidazo[1,2-a]pyridine-3-acetonitrile

| A. FORMULA | mg/supp |
|---|---|
| 8-Benzyloxy-3-cyanomethyl-2-methyl-imidazo[1,2-a]pyridine | 5.0 |
| Cocoa butter | 1.995.0 |
| | 2.0 g |

Procedure

1. Melt cocoa butter to about 32—35°C.
2. Blend the active ingredient into cocoa butter until well dispersed.
3. Pour into teflon-coated mold and congeal in refrigerator. Keep in refrigerator for an appropriate length of time.
4. Remove suppositories from mold.

| B. FORMULA | mg/supp |
|---|---|
| 8-Benzyloxy-3-cyanomethyl-2-methyl-imidazo[1,2-a]pyridine | 100.0 |
| PEG 1000 | 1.824.0 |
| PEG 4000 | 76.0 |
| | 2.0 g |

Procedure

1. Melt PEG 1000 and PEG 4000 in one container to 50°C.
2. Add the active ingredient to the mixture. Blend until well dispersed.
3. Pour into mold and congeal in refrigerator. Keep in refrigerator for an appropriate length of time.
4. Remove suppositories from mold.

As is obvious to anyone skilled in the art, the active ingredient used in the formulations above may be replaced by other compounds of this invention.

## Claims

1. Imidazo[1,2-a]pyridines of the general formula

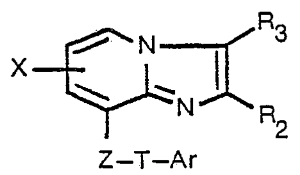 I

wherein

$R_2$ represents hydrogen, $C_1$ to $C_6$-loweralkyl, trifluoromethyl, B—$CF_3$; B—Ar, Ar, B-halogen, $BOR_8$, B—$SO_{(n)}$-($C_1$ to $C_6$-lower-alkyl), B—O—CO—$R_1$,

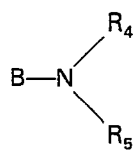, $COOR_6$, 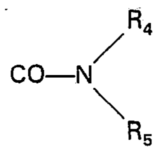, or BCN;

$R_3$ represents $C_1$ to $C_6$-loweralkyl, BCN, $BOR_8$, B—O—CO—$R_1$,

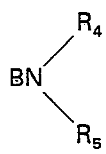, 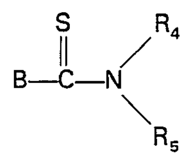, 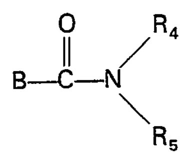,

B—$NO_2$, B-halogen or provided $R_2$ is not hydrogen, hydrogen;

X is hydrogen, $C_1$ to $C_6$-loweralkyl, halogen, hydroxy, trifluoromethyl or $C_1$ to $C_6$-loweralkoxy;

Z represents O, S, SO, $SO_2$, $NR_6$ or a single bond between the group T—Ar and position 8 of the imidazo[1,2-a]pyridine nucleus;

T represents a single bond between Z and Ar or a branched or straight alkylene group having 1 to 12 carbon atoms with no more than 5 binding carbon atoms between Z and Ar; and Ar represents phenyl, 2-, 3- or 4-pyridyl, 2- or 3-thienyl, 2- or 3-furanyl, 2- or 4-imidazolyl, whereby the pyridyl-; thienyl-; furanyl- and imidazolyl- groups may be substituted by halogen or $C_1$ to $C_6$-loweralkyl, or X'-, Y'- and/or Z'-substituted phenyl wherein each of X', Y' and Z' independently is hydrogen, halogen, $C_1$ to $C_6$-loweralkyl, $C_1$ to $C_6$-loweralkoxy, OH, $NO_2$, CN, $CF_3$,

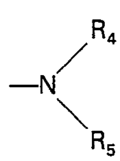, $SO_{(m)}$-$R_1$, 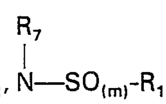

with m being zero, one or two whereby m represents two when $R_1$ represents

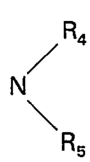), $COOR_6$, 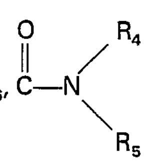 or , 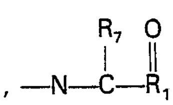

whereby in the above definitions

B is a straight or branched chain $C_1$ to $C_6$-lower alkylene group;

n represents zero, one or two;

$R_4$ and $R_5$ each independently represent hydrogen, $C_1$ to $C_6$-loweralkyl $C_1$ to $C_6$ loweralkoxy-($C_1$ to $C_6$ loweralkyl, trifluoromethyl-($C_1$ to $C_6$-loweralkyl), Ar-($C_1$ to $C_6$-loweralkyl), Ar or, when taken together with the nitrogen to which they are attached, form a 4- to 6-membered cyclic amino- group;

$R_1$ represents Ar-, $C_1$ to $C_6$-loweralkyl,

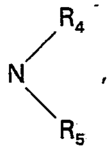 , 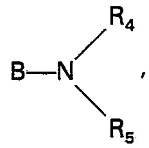 ,

or Ar-($C_1$ to $C_6$-loweralkyl)

$R_6$ represents hydrogen, $C_1$- to $C_{12}$-alkyl, aryl or an aralkyl group having up to 12 carbon atoms;

$R_7$ represents hydrogen or $C_1$ to $C_6$-loweralkyl; and

$R_8$ represents hydrogen, $C_1$ to $C_6$-loweralkyl, $C_1$ to $C_6$-loweralkoxy-($C_1$ to $C_6$-loweralkyl), trifluoromethyl-($C_1$ to $C_6$-loweralkyl), Ar-($C_1$ to $C_6$-loweralkyl) or Ar;

the 2,3-dihydro-5,6,7,8-tetrahydro- and 2,3,5,6,7,8-hexahydro-analogues thereof and the pharmaceutically acceptable salts of such compounds.

2. Compounds according to claim 1 wherein, in Formula I

$R_2$ represents hydrogen, loweralkyl with 1 to 3 carbon atoms, $CH_2OH$, $CH_2CN$, $CH_2O—CO—CH_2N(CH_3)_2$ or $CH_2—\underset{\underset{\textstyle O_{(n)}}{\|}}{S}—CH_3$

with n being zero, one or two;

$R_3$ is selected from $CH_3$, $CH_2CN$, $\overset{\overset{\textstyle C_2H_5}{|}}{C}H—CN$, $CH_2OH$, $CH_2\overset{\overset{\textstyle S}{\|}}{C}NH_2$, $CH_2—O—CO—R_9$

(with $R_9$ representing methyl, ethyl, propyl, isopropyl or t-butyl) and, provided $R_2$ is not hydrogen, hydrogen;

X represents hydrogen;

Z represents O, NH, SO or a single bond;

T represents a branched or straight lower alkylene group having up to 5 carbon atoms, and

Ar is selected from phenyl, thienyl, pyridyl or a X'-, Y'-, Z'-substituted phenyl group wherein each of X', Y' and Z' independently is selected from H, Cl, F, $CH_3$, t-butyl, $CF_3$, $OCH_3$ and CN.

3. Compounds according to claim 2 wherein in Formula I

$R_2$ represents H, $CH_3$, $C_2H_5$, $i$-$C_3H_7$ or $CH_2OH$;

$R_3$ represents $CH_3$, $CH_2CN$, $CH_2OH$, $CH_2OCOCH_3$ or $CH_2—\overset{\overset{\textstyle S}{\|}}{C}NH_2$;

Z represents —O, NH or a single bond;

T represents $—CH_2—$, $—CH_2—CH_2—$, $—\overset{\overset{\textstyle CH_3}{|}}{C}H—$ or $—CH_2—CH_2—CH_2—$;

and

Ar represents phenyl, o- or p-fluorophenyl, p-chlorophenyl, or 2,4,6-trimethylphenyl.

4. Compounds according to claim 3, namely
8-benzyloxy-3-cyanomethyl-2-methyl-imidazo[1,2-]pyridine;
8-benzyloxy-3-hydroxymethyl-2-methyl-imidazo[1,2-a]pyridine;
3-cyanomethyl-8-(2-fluorobenzyloxy)-2-methyl-imidazo[1,2-a]pyridine;
3-cyanomethyl-8-(4-fluorobenzyloxy)-2-methyl-imidazo[1,2-a]pyridine;
3-cyanomethyl-2-methyl-8-(2,4,6-trimethylbenzyloxy-imidazo[1,2-a]pyridine and
8-benzylamino-3-cyanomethyl-2-methyl-imidazo[1,2-a]pyridine.

5. Compounds according to claim 3, namely
3-cyanomethyl-2-methyl-8-(2-phenylethyl)-imidazo[1,2-a]pyridine;
8-benzyloxy-2-methyl-imidazo[1,2-a]pyridine-3-thioacetamide;
3-cyanomethyl-2-methyl-8-(3-phenylpropoxy)-imidazo[1,2-a]pyridine
8-benzylamino-2,3-dimethyl-imidazo[1,2-a]pyridine.
8-benzyloxy-2-methyl-imidazo[1,2-a]pyridine-3-methylacetate;
8-benzyloxy-2,3-dimethyl-imidazo[1,2-a]pyridine;
3-cyanomethyl-2-methyl-8-(2-phenylethoxy)-imidazo[1,2-a]pyridine and
3-cyanomethyl-2-methyl-8-(1-phenylethoxy)-imidazo[1,2-a]pyridine.

6. Compounds according to claim 3, namely
8-benzyloxy-3-cyanomethyl-imidazo[1,2-a]pyridine;

8-benzyloxy-3-cyanomethyl-2-isopropyl-imidazo[1,2-a]pyridine;
8-benzyloxy-3-cyanomethyl-2-ethyl-imidazo[1,2-a]pyridine and
8-benzyloxy-3-cyanomethyl-2-hydroxymethyl-imidazo[1,2-a]pyridine.

7. A compound according to claim 4 namely
8-benzyloxy-3-cyanomethyl-2-methyl-imidazo[1,2-a]pyridine.

8. Pharmaceutical compositions comprising as an active ingredient a compound as defined in any one of claims 1 to 7.

9. Process for preparing pharmaceutical compositions as defined in claim 8, characterized in that a compound as defined in any one of claims 1 to 7 is admixed with a suitable pharmaceutical carrier.

10. Process for preparing compounds of Formula I as defined in claim 1, characterized in that, either A: a compound of the general formula

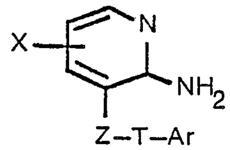

is reacted with a compound of the general Formula

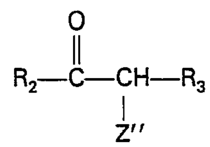

whereby in the formulae $R_2$, $R_3$, X, Z, T and Ar are as defined in claim 1, except that any free amino or hydroxy groups present in $R_2$, $R_3$, X or Ar may be protected by a protecting group which is subsequently removed, and Z″ represents a good leaving group; or, for preparation of compounds wherein Z is S, O, $NR_6$ or a single bond

B: a compound of the general Formula

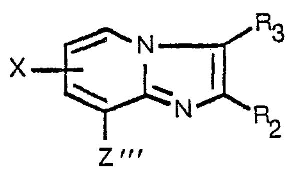

is reacted with a compound of the general Formula

Hal—T—Ar

whereby in the above formulae $R_2$, $R_3$, X, T and Ar are as defined in claim 1, Hal, represents Br, Cl or I and Z‴ represents hydrogen, halogen, OH, SH or $NHR_6$, with $R_6$ being as defined in claim 1; or that, for the preparation of compounds wherein $R_3$ represents BCN,

C: a compound of general Formula I wherein $R_2$, X, Z, T and Ar are as defined in claim 1 but wherein $R_3$ is replaced by a group BX″ wherein B is as defined in claim 1 and X″ represents a good leaving group, is reacted with a metal cyanide; or

D: a compound of Formula I wherein $R_2$, X, T, Z and Ar as defined in claim 1, but wherein $R_3$ is replaced by a group $BCONH_2$ or B—CH(=N—OH) is subjected to a dehydration; or

E: a compound of Formula I wherein $R_2$, X, T, Z and Ar as defined in claim 1, but $R_3$ is substituted by a group B—CHO, is treated with a suitable cyanide whereby the formyl function is replaced by CN; or

F: a compound of Formula I wherein $R_2$, X, T, Z and Ar are as defined in claim 1 but wherein $R_3$ is replaced by the group BCOOR, is treated with a suitable amine; or

G: a compound of Formula I wherein $R_2$, S, T, Z and Ar are as defined claim 1 but wherein $R_3$ is replaced by the group BCH(NH$_2$)—COOH is reacted with NaOCl; or

H: a compound of Formula I wherein $R_2$, X, Z, T and Ar are as defined in claim 1 but wherein $R_3$ is replaced by the group $BCH_2NO_2$ is subjected to a reductive dehydration; or

I: a compound of Formula I wherein $R_2$, X, T, Z and Ar are as defined in claim 1, and $R_3$ is hydrogen, is reacted with a compound hal-BCN, with hal representing chlorine or bromine in the presence of a Lewis acid; or

J: a compound of Formula I, wherein $R_2$, X, T, Z and Ar are as defined in claim 1 but wherein $R_3$ is replaced by the group 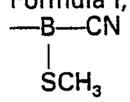

is subjected to a reduction replacing the $SCH_3$-group by hydrogen, and that a compound obtained according to either of process A to J above is, if desired, transformed into a pharmaceutically accetable salt.

11. Process according to claim 10, characterized in that in process

A: Z″ represents halogen, tosyl or mesyl and the reaction is carried out by heating the reactants together in an inert solvent;

C: X″ represents $Si(CH_3)_2$, halogen, alkoxy, aryloxy, mesyl, tosyl, a quaternary group, preferably $N^{\oplus}(CH_3)_3 \cdot I^{\ominus}$, or a quaternary group wherein the quaternary ion is a non-nucleophilic counter ion selected from $BF_4^{\ominus}$, $PF_6^{\ominus}$, $CF_3SO_3^{\ominus}$ and $FSO_3^{\ominus}$ and the metal cyanide is an alkali metal cyanide;

D: the hydration is carried out by using $(CF_3CO)_2O$, $SeO_2$ or $POCl_3$ as dehydrating agent;

E: Tosyl-$CH_2CN$ is used as cyanocompound and the reaction is carried out in the presence of potassium-t-butoxyde;

F: dimethylaluminium-amine is used as a suitable amine;

H: the reductive dehydration is carried out by means of $PCl_3$;

I: the reaction is carried out in the presence of a Lewis acid, preferably aluminium chloride, Zinc chloride or boron chloride;

J: the reduction is carried out by means of Raney-nickel.

**Patentansprüche**

1. Imidazo[1,2-a]pyridine der allgemeinen Formel

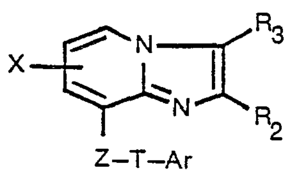

I

in der

$R_2$ Wasserstoff, $C_1$- bis $C_6$-Niederalkyl, Trifluoromethyl, B—$CF_3$, B—Ar, Ar, B—Halogen, $BOR_8$m, B—$SO_{(n)}$-($C_1$-bis $C_6$-Niederalkyl), B—O—CO—$R_1$

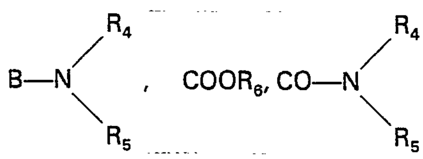

oder BCN bezeichnet;

$R_3$ $C_1$-bis $C_6$-Niederalkyl, BCN, $BOR_8$, B—O—CO—$R_1$,

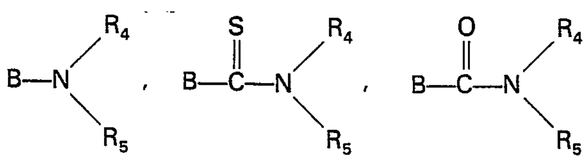

$BNO_2$, B—Halogen oder, falls $R_2$ nicht Wasserstoff ist, Wasserstoff bezeichnet;

X Wasserstoff, $C_1$- bis $C_6$-Niederalkyl, Halogen, Hydroxy, Trifluoromethyl oder $C_1$- bis $C_6$-Niederalkoxy ist;

Z O, S, SO, $SO_2$, $NR_6$ oder eine Einfachbindung zwischen der Gruppe T—Ar und der 8-Stellung des Imidazo[1,2-a]pyridin-Kerns bezeichnet;

T eine Einfachbindung zwischen Z und Ar oder eine verzweigte oder geradkettige Alkylf-Gruppe mit 1 bis 12 Kohlenstoff-Atom un mit nicht mehr als 5 bindenen Kohlenstoff-Atomen zwischen Z und Ar bezeichnet; und

Ar Phenyl, 2-, 3- oder 4-Pyridyl, 2- oder 3-Thienyl, 2- oder 3-Furanyl, 2- oder 4-Imidazolyl, wobei die Pyridyl-, Thienyl-, Furanyl- und Imidazolyl-Gruppen durch Halogen oder $C_1$- bis $C_6$-Niederalkyl substituiert sein können, oder X'-, Y'- und/oder Z'-substituiertes Phenyl bezeichnet, worin X', Y' und Z' unabhängig voneinander Wasserstoff,

Halogen, $C_1$- bis $C_6$-Niederalkyl, $C_1$- bis $C_6$-Niederalkoxy, OH, $NO_2$, CN, $CF_3$,

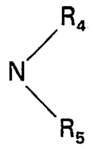 , $SO_{(m)}$-$R_1$, 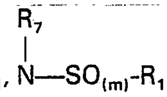

(worin m null, eines oder zwei ist, wobei m zwei ist, wenn $R_1$

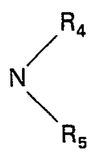 ), $COOR_6$, 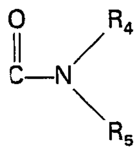 , oder 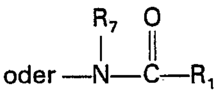

bezeichnet,

wobei in den vorstehenden Definitionen

B eine geradkettige oder kettenverzweigte $C_1$- bis $C_6$-Niederalkylen-Gruppe ist;

n null, eins oder zwei bezeichnet;

$R_4$ und $R_5$ unabhängig voneinander Wasserstoff, $C_1$- bis $C_6$-Niederalkyl, $C_1$- bis $C_6$-Niederalkoxy-($C_1$ bis $C_6$-Niederalkyl) Trifluoromethyl-($C_1$- bis $C_6$-Niederalkyl), Ar-($C_1$ bis $C_6$Niederalkyl), Ar bezeichnen oder, wenn sie mit dem Stickstoff, an den sie gebunden sind, zusammen genommen werden, eine 4- bis 6-gliedrige cyclische Amino-Gruppe bilden;

$R_1$ Ar-, $C_1$- bis $C_6$-Niederalkyl,

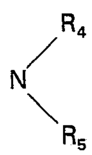 , 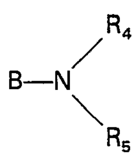

oder Ar-($C_1$-bis $C_6$-Niederalkyl) bezeichnet;

$R_6$ Wasserstoff, $C_6$- bis $C_{12}$-Alkyl, Aryl oder eine Aralkyl-Gruppe mit bis zu 12 Kohlenstoff-Atomen bezeichnet;

$R_7$ Wasserstoff oder $C_1$- bis $C_6$-Niederalkyl bezeichnet und

$R_8$ Wasserstoff, $C_1$- bis $C_6$-Niederalkyl, $C_1$- bis $C_6$-Niederalkoxy-($C_1$- bis $C_6$-Niederalkyl), Trifluoromethyl-($C_1$- bis $C_6$-Niederalkyl), Ar-($C_1$- bis $C_6$-Niederalkyl) oder Ar bezeichnet, deren 2,3-Dihydro-5,6,7,8-tetrahydro- und 2,3,5,6,7,8-Hexahydro-Analoga sowie pharmazeutisch unbedenkliche Salze derartiger Verbindungen.

2. Verbindungen nach Anspruch 1, dadurch gekennzeichnet, daß in der Formel I

$R_2$ Wasserstoff, Niederalkyl mit 1 bis 3 Kohlenstoff-Atomen, $CH_2OH$, $CH_2CN$, $CH_2O$—CO—$CH_2N(CH_3)_2$ oder 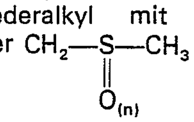

bezeichnet, wobei n null, eins oder zwei ist;

$R_3$ ausgewählt ist aus $CH_3$, $CH_2CN$, 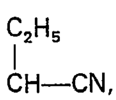, $CH_2OH$, 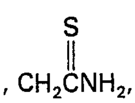, $CH_2$—O—CO—$R_9$

(wobei $R_9$ Methyl, Ethyl, Propyl, Isopropyl oder t-Butyl bezeichnet) und, falls $R_2$ nicht Wasserstoff ist, Wasserstoff;

X Wasserstoff bezeichnet;

Z O, NH, SO oder eine Einfachbindung bezeichnet;

T eine verzweigte oder geradkettige niedere Alkylen-Gruppe mit bis zu 5 Kohlenstoff-Atomen bezeichnet und

Ar ausgewählt ist aus Phenyl, Thienyl, Pyridyl oder einer X'-, Y'-, Z'-substituierten Phenyl-Gruppe,

worin jeweils X′, Y′ und Z′ unabhängig voneinander ausgewählt sind aus H, Cl, F, $CH_3$, t-Butyl, $CF_3$, $OCH_3$ und CN.

3. Verbindungen nach Anspruch 2, dadurch gekennzeichnet, daß in der Formel I
$R_2$ H, $CH_3$, $C_2H_5$, i-$C_3H_7$ oder $CH_2OH$ bezeichnet;
$R_3$ $CH_3$, $CH_2CN$, $CH_2OH$, $CH_2OCOCH_3$ oder 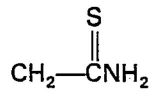
bezeichnet;
Z —O, NH, oder eine Einfachbindung bezeichnet;
T —$CH_2$—, —$CH_2$—$CH_2$—, 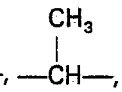, —$CH_2$—$CH_2$—$CH_2$— bezeichnet und
Ar Phenyl, o- oder p-Fluorophenyl, p-Chlorophenyl oder 2,4,6-Trimethylphenyl bezeichnet.

4. Verbindungen nach Anspruch 3, nämlich 8-Benzyloxy-3-cyanomethyl-2-methyl-imidazo[1,2-a]pyridin;
8-Benzyloxy-3-hydroxymethyl-2-methyl-imidazo[1,2-a]pyridin;
3-Cyanomethyl-8-(2-fluorobenzyloxy)-2-methyl-imidazo[1,2-a]pyridin;
3-Cyanomethyl-8-(4-fluorobenzyloxy)-2-methyl-imidazo[1,2-a]pyridin;
3-Cyanomethyl-2-methyl-8-(2,4,6-trimethylbenzyloxy)-imidazo[1,2-a]pyridin und
8-Benzylamino-3-cyanomethyl-2-methyl-imidazo[1,2-a]pyridin.

5. Verbingunden nach Anspruch 3, nämlich 3-Cyanomethyl-2-methyl-8-(2-phenylethyl)-imidazo[1,2-a]pyridin;
8-Benzyloxy-2-methyl-imidazo[1,2-a]pyridin-3-thioacetamid;
3-Cyamomethyl-2-methyl-8-(3-phenylpropoxy)-imidazo[1,2-a]pyridin;
8-Benzylamino-2,3-dimethyl-imidazo[1,2-a]pyridin;
8-Benzyloxy-2-methyl-imidazo[1,2-a]pyridin-3-methylacetat;
8-Benzyloxy-2,3-dimethyl-imidazo[1,2-a]pyridin;
3-Cyanomethyl-2-methyl-8-(2-phenylethoxy)-imidazo[1,2-a]pyridin und
3-Cyanomethyl-2-methyl-8-(1-phenylethoxy)-imidazo[1,2-a]pyridin.

6. Verbindungen nach Anspruch 3, nämlich
8-Benzyloxy-3-cyanomethyl-imidazo[1,2-a]pyridin;
8-Benzyloxy-3-cyanomethyl-2-isopropyl-imidazo[1,2-a]pyridin;
8-Benzyloxy-3-cyanomethyl-2-ethyl-imidazo[1,2-a]pyridin und
8-Benzyloxy-3-cyanomethyl-2-hydroxymethyl-imidazo[1,2-a]pyridin.

7. Verbindung nach Anspruch 4, nämlich
8-Benzyloxy-3-cyanomethyl-2-methyl-imidazo[1,2-a]pyridin.

8. Pharmazeutische Zusammensetzungen, enthaltend als Wirkstoff eine Verbindung nach irgendeinem der Ansprüche 1 bis 7.

9. Verfahren zur Herstellung pharmazeutischer Zusammensetzungen nach Anspruch 8, dadurch gekennzeichnet, daß eine Verbindung nach irgendeinem der Ansprüche 1 bis 7 mit einem geeigneten pharmazeutischen Träger vermischt wird.

10. Verfahren zur Herstellung von Verbindungen der Formel I nach Anspruch 1, dadurch gekennzeichnet, daß entweder
A: eine Verbindung der allgemeinen Formel

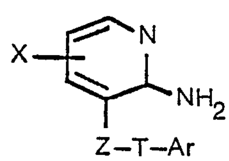

mit einer Verbindung der allgemeinen Formel

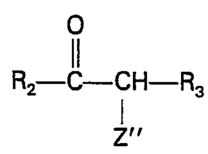

zur Reaktion gebracht wird, wobei in den Formeln $R_2$, $R_3$, X, Z, T und Ar die in Anspruch 1 angegebenen Bedeutungen haben, jedoch mit der Ausnahme, daß in $R_2$, $R_3$, X oder Ar etwa vorhandene freie Amino- oder Hydroxy-Gruppen durch Schutzgruppen geschützt sein können, die anschließend entfernt werden; und Z″ eine liecht abspaltbare Gruppe bezeichnet; oder

zur Herstellung von Verbindungen, in denen Z S, O, $NR_6$ oder eine Einfachbindung ist,

B: eine Verbindung der allgemeinen Formel

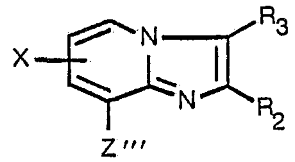

mit einer Verbindung der allgemeinen Formel

Hal—T—Ar

zur Reaktion gebracht wird, wobei in den vorstehenden Formeln $R_2$, $R_3$, X, T und Ar die in Anspruch 1 angegebenen Bedeutungen haben, Hal, Br, Cl oder I bezeichnet und Z‴ Wasserstoff, Halogen, OH, SH oder $NHR_6$ bezeichnet, wobei $R_6$ die in Anspruch 1 angegebene Bedeutung hat; oder daß zur Herstellung von Verbindungen in denen $R_3$ BCN bezeichnet.

C: eine Verbindung der allgemeinen Formel I, in der $R_2$, X, Z, T und Ar die in Anspruch 1 angegebenen Bedeutungen haben, in der jedoch $R_3$ durch eine Gruppe BX″ ersetzt ist, worin B die in Anspruch 1 angegebene Bedeutung hat und X″ eine leicht abspaltbare Gruppe bezeichnet, mit einem Metallcyanid zur Reaktion gebracht wird; oder

D: eine Verbindung der allgemeinen Formel I, in der $R_2$, X, T, Z und Ar die in Anspruch 1 angegebenen Bedeutungen haben, in der jedoch $R_3$ durch eine Gruppe $BCONH_2$ oder $B\text{—}CH{=}N\text{—}OH$ ersetzt ist, einer Wasserabspaltung unterworfen wird; oder

E: eine Verbindung der allgemeinen Formel I, in der $R_2$, X, T, Z und Ar die in Anspruch 1 angegebenen Bedeutungen haben, in der jedoch $R_3$ durch eine Gruppe B—CHO ersetzt ist, mit einem geeigneten Cyanid behandelt wird, woduch die Formyl-Funktion durch CN ersetzt wird; oder

F: eine Verbindung der allgemeinen Formel I, in der $R_2$, X, T, Z und Ar die in Anspruch 1 angegebenen Bedeutungen haben, in der jedoch $R_3$ durch eine Gruppe BCOOR ersetzt ist, mit einem geeigneten Amin behandelt wird; oder

G: eine Verbindung der allgemeinen Formel I, in der $R_2$, X, T, Z und Ar die in Anspruch 1 angegebenen Bedeutungen haben, in der jedoch $R_3$ durch eine Gruppe $BCH(NH_2)\text{—}COOH$ ersetzt ist, mit NaOCl zur Reaktion gebracht wird; oder

H: eine Verbindung der allgemeinen Formel I, in der $R_2$, X, Z, T und Ar die in Anspruch 1 angegebenen Bedeutungen haben, in der jedoch $R_3$ durch eine Gruppe $BCH_2NO_2$ ersetzt ist, einer reduzierenden Wasserabspaltung unterworfen wird; oder

I: eine Verbindung der allgemeinen Formel I, in der $R_2$, X, T, Z und Ar die in Anspruch 1 angegebenen Bedeutungen haben und $R_3$ Wasserstoff ist, mit einer Verbindung hal—BCN, worin hal Chlor oder Brom bezeichnet, in Gegenwart einer Lewis-Säure zur Reaktion gebracht wird; oder

J: eine Verbindung der allgemeinen Formel I, in der $R_2$, X, T, Z und Ar die in Anspruch 1 angegebenen Bedeutungen haben, in der jedock $R_3$ durch eine Gruppe $\text{—}BCN(SCH_3)$ ersetzt ist, einer Reduktion unterworfen wird, wodurch die $SCH_3$—Gruppe durch Wasserstoff ersetzt wird;

und daß eine nach einem der vorstehenden Verfahren A bis J erhaltene Verbindung gewünschtenfalls in ein pharmazeutisch unbedenkliches Salz überführt wird.

11. Verfahren nach Anspruch 10, dadurch gekennzeichnet, daß daß in Verfahren

A: Z″ Halogen, Tosyl oder Mesyl bezeichnet und die Reaktion durch gemeinsames Erhitzen der Reaktions-partner in einen inerten Lösungsmittel durchgeführt wird;

C: X″ $Si(CH_3)_2$, Halogen, Alkoxy, Aryloxy, Mesyl, Tosyl, eine quaternäre Gruppe, vorzugsweise $N^{\oplus}(CH_3)_3.I^{\ominus}$ oder eine solche quaternäre Gruppe bezeichnet, in der das quaternäre Ion ein nicht-nucleophiles Gegenion ausgewählt aus $BF_4^{\ominus}$, $PF_6^{\ominus}$, $CF_3SO_3^{\ominus}$ und $FSO_3^{\ominus}$ ist, und das Metallcyanid ein Alkalimetallcyanid ist;

D: die Wasserabspaltung durch Verwendung von $(CF_3CO)_2O$, $SeO_2$ oder $POCl_3$ als wasserabspaltendes Mittel durchgeführt wird;

E: Tosyl-$CH_2$—CN als Cyan-Verbindung verwendet wird und die Reaktion in Gegenwart von kalium-t-butoxid durchgeführt wird;

F: Dimethylaluminiuamin als geeignetes Amin verwendet wird;

H: die reduzierende Wasserabspaltung mit Hilfe von $PCl_3$ durchgeführt wird;

I: die Reaktion in Gegenwart einer Lewis-Säure, vorzugsweise Aluminiumchlorid, Zinkchlorid oder Borchlorid, durchgeführt wird;

J: die Redution mit Hilfe von Raney-Nickel durchgeführt wird.

**Revendications**

1. Imidazo[1,2-a]pyridines caractérisées en ce qu'elles ont pour formule générale:

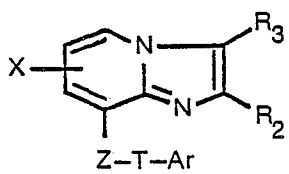

I

où

$R_2$ représente de l'hydrogène, un alcoyle inférieur $C_1$ à $C_6$, du trifluorométhyle, B—$CF_3$; B—Ar, Ar, B-halogène, $BOR_8$, B—$SO_{(n)}$-alcoyle inférieur $C_1$ à $C_6$, B—O—CO—$R_1$,

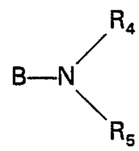 , $COOR_6$, 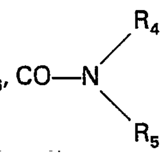

ou BCN;

$R_3$ représente un alcoyle inférieur $C_1$ à $C_6$, BCN, $BOR_8$, B—O—CO—$R_1$,

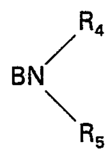 , 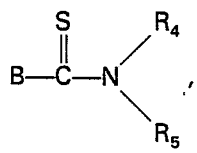 , 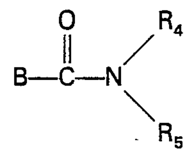

B—$NO_2$, B-halogène ou, à condition que $R_2$ ne soit pas de l'hydrogène, de l'hydrogène;

X est de l'hydrogène, un alcoyle inférier $C_1$ à $C_6$, un halogène, un hydroxy, un trifluorométhyle ou un alcoxy inférieur $C_1$ à $C_6$;

Z représente O, S, SO, $SO_2$, $NR_6$ ou une simple liaison entre le groupe T—Ar et la position 8 du noyau imidazo[1,2-a]pyridine;

T représente une simple liaison entre Z et Ar ou un groupe alcoylène droit ou ramifié ayant 1 à 12 atomes de carbone avec pas plus de 5 atomes de carbone de liaison entre Z et Ar; et

Ar représente du phényle, du 2-, 3- ou 4-pyridyle, due 2- ou 3-thiényle, du 2- ou 3-furanyle, du 2- ou 4-imidazolyle, les groupes pyridyle, thiényle, furanyle et imidazolyle pouvant être substitués par un halogène ou un alcoyle inférieur $C_1$ à $C_6$, ou un phényle substitué en X'-, Y'- et/ou Z'-, où chacun de X', Y' et Z' est indépendamment de l'hydrogène, un halogène, un alcoyle inférieur $C_1$ à $C_6$, un alcoxy inférieur $C_1$ à $C_6$, OH, $NO_2$, CN, $CF_3$,

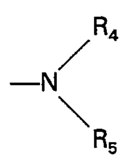 , $SO_{(m)}$—$R_1$, 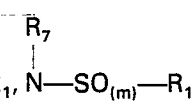

(m étant zéro, un deux, et m représente deux quand $R_1$ représente

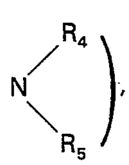, $COOR_6$, 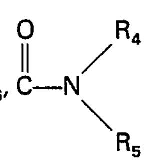 ou 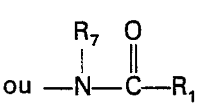

dans les définitions ci-dessus,

B est un groupe alcoylène $C_1$ à $C_6$ à chaîne droite ou ramifiée,

n représente zéro, un ou deux,

$R_4$ et $R_5$ représentent chacun indépendamment de l'hydrogène un alcoyle inférieur $C_1$ à $C_6$, un alcoxy inférier $C_1—C_6$-(alcoyle inférieur $C_1—C_6$), un trifluorométhyl (alcoyle inférieur $C_1—C_6$), Ar (alocyle inférieur $C_1—C_6$), Ar, ou, en les prenant ensemble avec l'azote auquel ils sont attachés, ils forment un groupe amino cyclique à 4 à 6 éléments;

$R_1$ représente Ar, un alcoyle inférier $C_1$ à $C_6$,

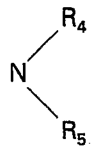 , 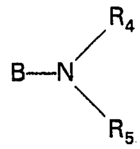

ou Ar (alcoyle inférieur $C_1—C_6$);

$R_6$ représente de l'hydrogène, un alcoyle de 1 à 12 atomes de carbone, un aryle ou un groupe aralcoyle ayant jusqu'à 12 atomes de carbone;

$R_7$ représente de l'hydrogène ou un alcoyle inférieur $C_1—C_6$; et

$R_8$ représente de l'hydrogène, un alcoyle inférieur $C_1—C_6$, un alcoxy inférieur $C_1—C_6$-(alcoyle inférieur $C_1—C_6$), un trifluorométhyl(alcoyle inférieur $C_1—C_6$), Ar-(alcoyle inférieur $C_1—C_6$) ou Ar;

les analogues 2,3-dihydro-5,6,7,8-tétrahydro et 2,3,4,5,6,7,8-hexahydro et les sels acceptables en pharmacie de tels composés.

2. Composés selon la revendication 1, caractérise en ce que, dans la formule I,

$R_2$ représente de l'hydrogène, un alcoyle inférieur de 1 à 3 atomes de carbone, $CH_2OH$, $CH_2CN$, $CH_2O—CO—CH_2N(CH_3)_2$ ou $CH_2—\underset{\underset{O_{(n)}}{\|}}{S}—CH_3$;

n étant zéro, un ou deux,

$R_3$ est choisi parmi

$$CH_3,\ CH_2CN,\ \overset{\overset{C_2H_5}{|}}{CH}—CN,\ CH_2OH,\ CH_2\overset{\overset{S}{\|}}{C}NH_2,\ CH_2—O—CO—R_9$$

($R_9$ représentant du méthyle, de l'éthyle, du propyle, de l'isopropyle ou du t-butyle) et, à condition que $R_2$ ne soit pas de l'hydrogène, de l'hydrogène;

X représente de l'hydrogène;

Z représente O, NH, SO ou une simple liaison;

T représente un groupe alcoylène inférier à chaîne droite ou ramifiée ayant jusqu'à 5 atomes de carbone et

Ar est choisi parmi le phényle, le thiényle, le pyridyle ou un groupe phényle substitué en X'-, Y'-, Z'-, oỳ chacun de X', Y' et Z' est indépendamment choisi parmi H, Cl, F, $CH_3$, du t-butyle $CF_3$, $OCH_3$ et CN.

3. Composés selon la revendication 2, caractérisés en ce que dans la formule I

$R_2$ représente H, $CH_3$, $C_2H_5$, $i\text{-}C_3H_7$ ou $CH_2OH$;

$R_3$ représente $CH_3$, $CH_2CN$, $CH_2OH$, $CH_2OCOCH_3$ ou $CH_2—\overset{\overset{S}{\|}}{C}NH_2$;

Z représente —O, NH ou une simple liaison;

T représente $—CH_2—$, $—CH_2—CH_2—$, $—\overset{\overset{CH_3}{|}}{CH}—$ ou $—CH_2—CH_2—CH_2$; et

Ar représente du phényle, du o- ou p-fluorophényle, du p-chlorophényle, ou du 2,4,6-triméthylphényle.

4. Composés selon la revendication 3, caractérisés en ce qu'il sont représentés par

8-benzyloxy-3-cyanométhyl-2-méthyl-imidazo[1,2-a]pyridine;

8-benzyloxy-3-hydroxyméthyl-2-méthyl-imidazo[1,2-a]pyridine;

3-cyanométhyl-8-(2-fluorobenzyloxy)-2-méthyl-imidazo[1,2-a]pyridine;

3-cyanométhyl-8-(4-fluorobenzyloxy)-2-méthyl-imidazo[1,2-a]pyridine;

3-cyanométhyl-2-méthyl-8-(2,4,6-triméthyl benzyloxy-imidazo[1,2-a]pyridine et

8-benzylamino-3-cyanométhyl-2-méthyl-imidazo[1,2-a]pyridine.

5. Composés selon la revendication 3, caractérisés en ce qu'ils sont représentés par:

3-cyanométhyl-2-méthyl-8-(2-phényléthyl)-imidazo[1,2-a]pyridine;

8-benzyloxy-2-méthyl-imidazo[1,2-a]pyridine-3-thio--acétamide;

3-cyanométhyl-2-méthyl-8-(3-phénylpropoxy)-imidazo[1,2-a]pyridine;

8-benzylamino-2,3-diméthyl-imidazo[1,2-a]pyridine;

8-benzyloxy-2-méthyl-imidazo[1,2-a]pyridine-3-méthylacétate;

8-benzyloxy-2,3-diméthyl-imidazo[1,2-a]pyridine;
3-cyanométhyl-2-méthyl-8-(2-phényléthoxy)-imidazo[1,2-a]pyridine et
3-cyanométhyl-2-méthyl-8-(1-phényléthoxy)-imidazo[1,2-a]pyridine.

6. Composés selon la revendication 3, caractérisés en ce qu'ils sont repréentés par:
8-benzyloxy-3-cyanométhyl-imidazo[1,2-a]]pyridine;
8-benzyloxy-3-cyanométhyl-2-isopropyl-imidazo[1,2-a]pyridine;
8-benzyloxy-3-cyanométhyl-2-éthyl-imidazo[1,2-a]pyridine et
8-benzyloxy-3-cyanométhyl-2-hydroxyméthyl-imidazo[1,2-a]pyridine.

7. Composé selon la revendication 4, caractérisé en ce que c'est une 8-benzyloxy-3-cyanométhyl-2-méthyl-imidazo[1,2-a]pyridine.

8. Compositions pharmaceutiques caractérisées en ce qu'elles contiennent comme ingrédient actif, un composé selon l'une quelconque des revendication 1 à 7.

9. Procédé de préparation d'une composition pharmaceutique selon la revendication 8, caractérisé en ce qu'un composé selon l'une quelconque des revendications 1 à 7 mélangé à un véhicule pharmaceutique approprié.

10. Procédé de préparation des composés de formule I selon la revendication 1, caractérisé en ce que, soit:

A: on fait réagir un composé de formule généale

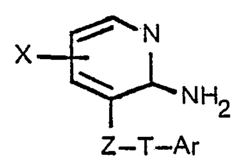

avec un composé de formule générale

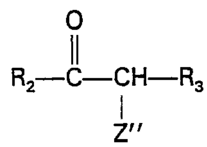

dans les formules, $R_2$, X, Z, T et Ar sont tels que définis à la revendication 1, à l'exception que tout groupe amino ou hydroxy libre présent dans $R_2$, $R_3$, X ou Ar peut être protégé par un groupe protecteur qui est subséquemment retiré, et Z'' représente un groupe partant; ou pour la préparation de composés où Z est S, O, $NR_6$ ou une simple liaison

B: on fait réagir un composé de formule générale

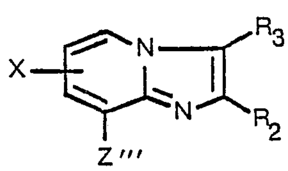

avec un composé de formule générale

Hal—T—Ar

dans les formules ci-dessus, $R_2$, $R_3$, X, T et Ar sont tels que définis à la revendication 1, Hal représente Br, Cl ou I et Z''' représente de l'hydrogène, un halogène, OH, SH ou $NHR_6$, $R_6$ étant tel que défini 7 la revendication 1; ou

pour la préparation de composés où $R_3$ représente BCN,

C: on fait réagir un composé de formule générale I où $R_2$, X, Z, T et Ar sont tels que définis à la revendication 1 mais où $R_3$ est remplacé par un groupe BX'' où B est tel que défini à la revendication 1 et X'' représente un groupe partant, avec un cyanure de métal; ou

D: un composé de formule I où $R_2$, X, T, Z et Ar sont tels que définis à la revendication 1, mais où $R_3$ est remplacé par un groupe $BCONH_2$ ou 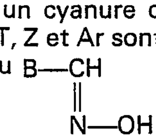
est soumis à une déshydration; ou

E: un composé de formule I où $R_2$, X, T, Z et Ar sont tels que définis à la revendication 1, mais $R_3$ est substitué par un groupe B—CHO, est traité avec un cyanure approprié où la fonction formyle est remplacée par CN; ou

F: un composé de formule I où $R_2$, X, T, Z et Ar sont tels que définis à la revendication 1 mais où $R_3$ est remplacé par le groupe BCOOR, est traité avec une amine appropriée; ou

G: on fait réagir un composé de formule I où $R_2$, X, T, Z et Ar sont tels que définis à la revendication 1, mais où $R_3$ est remplacé par le groupe BCH—COOH,

$$\begin{array}{l} \mathrm{BCH{-}COOH,} \\ \ \ \ | \\ \ \ \ \mathrm{NH_2} \end{array}$$

avec NaOCl; ou

H: un composé de formule I où $R_3$, X, Z, T et Ar sont tels que définis à l revendication 1 mais où $R_3$ est remplacé par le groupe $BCN_2NO_2$ est soumis à une déshydratation réductrice; ou

I: on fait réagir un composé de formule I où $R_2$, X, T, Z et Ar sont tels que définis à la revendication 1, et $R_5$ est de l'hydrogène, avec un composé Hal-BCN, Hal représentant du chlore ou du brome, en présence d'un acide de Lewis, ou

J: un composé de formule I où $R_2$, X, T, Z et Ar sont tels que définis à la revendication 1 mais où $R_3$ est remplacé par le groupe —B—CN,

$$\begin{array}{l} \mathrm{{-}B{-}CN,} \\ \ \ \ | \\ \ \mathrm{SCH_3} \end{array}$$

est soumis à une réduction remplaçant le groupe —$SCH_3$ par de l'hydrogène, et en ce qu'un composé obtenu selon les procédés A à J ci-dessus, est, si on le souhaite, transformé en son sel acceptable en pharmacie.

11. Procédé selon la revendication 10, caractérisé en ce que dans les procédés

A: Z'' représente un halogène, du tosyle ou de mésyle et la réaction est effectuée en chauffant les réactifs ensemble dans un solvant inerte;

C: X'' représente $Si(CH_3)_2$, un halogène, un alcoxy, un aryloxy, un mésyle, un tosyle, un groupe quaternaire, de préférence $N^{\oplus}(CH_3).I^{\ominus}$, ou un groupe quaternaire où l'ion quaternaire est un contre-ion non nucléophile choisi parmi $BF_4^{\oplus}$, $PF_6^{\ominus}$, $CF_3SO_3^{\ominus}$ et $FSO_3^{\ominus}$ et le cyanure d'un métal est un cyanure d'un métal alcalin;

D: l'hydratation est effectuée en utilisant $(CF_3CO)_2O$, $SeO_2$ ou $POCl_3$ comme agent de déshydratation;

E: on utilise du tosyl-$CH_2$—CN comme composé cyano et la réaction est effectuée en présence de t-butoxyde de potassium;

F: on utilise de la diméthylaluminium-amine comme amine appropriée;

H: la déshydratation réductrice est effectuée au moyen de $PCl_3$;

I: la réaction est effectuée en présence d'un acide de Lewis, de préférence de chlorure d'aluminium, du chlorure de zinc et du chlorure de bore;

J: la réduction est effectuée au moyen de nickel de Raney.